(19)

Europäisches Patentamt

European Patent Office

Office européen des brevets

(11)  **EP 4 417 621 A1**

(12)  **EUROPEAN PATENT APPLICATION**
published in accordance with Art. 153(4) EPC

(43) Date of publication:
**21.08.2024 Bulletin 2024/34**

(21) Application number: **22880203.9**

(22) Date of filing: **08.10.2022**

(51) International Patent Classification (IPC):
**C07K 16/10** (2006.01)        **C12N 15/11** (2006.01)
**C12N 5/07** (2010.01)         **C07K 19/00** (2006.01)
**A61K 39/42** (2006.01)        **A61P 31/14** (2006.01)
**A61P 11/06** (2006.01)

(52) Cooperative Patent Classification (CPC):
**C07K 16/1003; A61K 39/42; A61K 47/68;
A61P 11/06; A61P 31/14; C07K 19/00; C12N 5/06;
C12N 5/10; C12N 15/11**

(86) International application number:
**PCT/CN2022/123876**

(87) International publication number:
**WO 2023/061264 (20.04.2023 Gazette 2023/16)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **12.10.2021 CN 202111189228**

(71) Applicant: **Center for Excellence in Molecular Cell
Science,
Chinese Academy of Sciences
Shanghai 200031 (CN)**

(72) Inventors:
• **SUN, Bing**
  **Shanghai 200031 (CN)**
• **YI, Chunyan**
  **Shanghai 200031 (CN)**
• **LING, Zhiyang**
  **Shanghai 200031 (CN)**

(74) Representative: **Santoro, Sofia et al
Società Italiana Brevetti S.p.A.
Via Giosuè Carducci, 8
20123 Milano (IT)**

Remarks:
The complete document including Reference Table(s) and the Sequence Listing(s) can be downloaded from the EPO website

(54)  **DESIGN AND APPLICATION OF FULLY HUMAN ANTIBODY FOR NEUTRALIZING RESPIRATORY SYNCYTIAL VIRUS**

(57)  The present invention provides a design and application of a fully human antibody for neutralizing respiratory syncytial virus. Specifically, in the present invention, germline gene back mutation and affinity enhancement are performed on respiratory syncytial virus fusion protein fully-human monoclonal antibody 4FI by using computer aided design and site-directed mutagenesis technology. The modified antibody of the present invention has a relatively low number of somatic mutation sites, has high affinity, can effectively prevent RSV infection, and can also effectively inhibit the spread of RSV infection.

EP 4 417 621 A1

**Description**

**TECHNICAL FIELD**

The present invention relates to the field of biomedicine, in particular to design and use of a fully human antibody for neutralizing the respiratory syncytial virus.

**BACKGROUND**

Respiratory syncytial virus (RSV) is the leading cause of lower respiratory tract infections in children. RSV infection causes 6.7% of deaths in children aged 1 month to 1 year due to severe bronchiolitis or pneumonia. RSV infects almost all children before the age of 2 years. In older populations, respiratory syncytial virus contributes to a disease burden comparable to that of seasonal influenza A viruses. Despite the significant threat to human health caused by RSV, there is still a lack of clinically licensed RSV vaccines and effective antiviral drugs.

The RSV F protein mediates the fusion process between the viral envelope and the host cell membrane through the conformation change from the pre-fusion state (pre-F, pre-fusion F) to the post-fusion state (post-F, post-fusion F), which is also the process of its structural change from the pre-fusion state to the fusion state. Due to its critical role in RSV invasion and high conservation, the RSV F protein is a target for neutralizing antibodies and a major antigen for vaccine development. Numerous studies have demonstrated that the recognition sites of highly active neutralizing antibody against F proteins are predominantly on pre-fusion F protein, and that recombinant proteins designed on the basis of pre-fusion F are significantly more immunogenic and protective than vaccines designed on the basis of post-fusion F proteins.

Over the past decades, several approaches have been investigated for the prevention and treatment of RSV infections, including vaccine development, antiviral compounds (Ribavirin), antisense drugs, RNA interference techniques, and antibody products such as immunoglobulins or intravenous monoclonal antibodies. Only Palivizumab has been approved for RSV prevention in high-risk children. However, there is no vaccine or commercially available treatment for RSV in China. Only ribavirin has been approved for the treatment of RSV infection, but it has serious side effects. Palivizumab targets the conserved region of the F protein and has a broad-spectrum effect. However, the current dose of palivizumab is 15 mg/kg (body weight) per administration, and passive immunization should be given 5 times a year, which has the disadvantages of high dose, multiple administrations, and expensive price. Furthermore, Palivizumab is a humanized antibody that still retains some of murine sequences, which may have some immunogenicity and is controversial in terms of safety. With the development of immunology and molecular biology, the rapid development of genetically engineered antibodies, chimeric antibodies, humanized antibodies, and fully human antibodies have been continuously developed to minimize or even eliminate the HAMA reaction. Fully human antibodies in particular have become the direction of the future development of antibody drugs. Important considerations for antibody-based therapies are whether the antibody has broad-spectrum antiviral effects, better physicochemical properties, and whether the antibody is effective enough to achieve reasonably feasible dose to mediate protection.

Therefore, there is a need in this field to develop a novel medicament candidate against RSV viral infection.

**SUMMARY OF THE INVENTION**

The purpose of the present invention is to provide a novel medicament candidate against RSV virus infection. Specifically, the present invention discloses the design of a fully human antibody for neutralizing respiratory syncytial virus, as well as the use of the optimized 4F1 antibody in the prevention and treatment of RSV-associated diseases, and the use thereof in the diagnosis of RSV infections.

In the first aspect of the present invention, it provides an antibody specific to respiratory syncytial virus fusion protein, wherein the antibody comprises a heavy chain and a light chain,

wherein, the variable region of the heavy chain comprises the following amino acid mutations corresponding to a sequence as shown in SEQ ID NO: 15:

(Z1) n reverse mutations selected from the following group, wherein n is 3, 4, 5, 6, 7, 8 or 9:

position 1, E→Q
position 6, Q→E
position 13, R→Q
position 62, E→D
position 65, R→K
position 69, S→T

position 76, T→K
position 88, P→A
position 119, R→T;

(Z2) a neutralization-enhanced FR mutation
position 75, S→R;
(Z3) a combination of Z1 and Z2; and
the variable region of the light chain comprises the following amino acid mutations corresponding to a sequence as shown in SEQ ID NO: 16:

(Y1) m reverse mutations selected from the following group, wherein m is 3, 4, 5, 6 or 7:

position 12, S→P
position 24, K→R
position 47, K→Q
position 65, A→D
position 79, E→K
position 109, V→L
position 110, D→E;

(Y2) a neutralization-enhanced CDR mutation
position 30, L→R;
(Y3) a combination of Y1 and Y2.

In another preferred embodiment, the heavy chain variable region has 4 to 10 of the amino acid mutations corresponding to SEQ ID NO: 15.

In another preferred embodiment, the light chain variable region has 4 to 8 of the amino acid mutations corresponding to SEQ ID NO: 16.

In another preferred embodiment, the antibody specifically binds to the respiratory syncytial virus pre-fusion F protein.

In another preferred embodiment, the mutation in the heavy chain variable region corresponding to SEQ ID NO: 15 is selected from the group consisting of: E1Q, Q6E, R13Q, E62D, R65K, S69T, S75R, T76K, P88A, R119T, and a combination thereof.

In another preferred embodiment, the heavy chain variable region is selected from the group consisting of:

a) a heavy chain variable region of which the amino acid sequence is shown in SEQ ID NO: 1 or 2; and
b) a heavy chain variable region as shown in a derived sequence of the amino acid sequence as shown in SEQ ID NO: 1 or 2, which is obtained by maintaining 3 CDR regions and substituting, deleting, modifying and/or adding at least one (e.g., 1-5, preferably 1-3, and preferably 1 or 2) amino acid residue in FR regions, and retains a binding affinity to the respiratory syncytial virus fusion protein (preferably the pre-fusion F protein).

In another preferred embodiment, the mutation in the light chain variable region corresponding to SEQ ID NO: 16 is selected from the group consisting of: S12P, K24R, L30R, K47Q, A65D, E79K, V109L, D110E, and a combination thereof.

In another preferred embodiment, the light chain variable region is selected from the group consisting of:

c) a light chain variable region of which the amino acid sequence is shown in SEQ ID NO: 4 or 5; and
d) a heavy chain variable region as shown in a derived sequence of the amino acid sequence as shown in SEQ ID NO: 4 or 5, which is obtained by maintaining 3 CDR regions and substituting, deleting, modifying and/or adding at least one (e.g., 1-4, preferably 1-3, and preferably 1 or 2) amino acid residue in FR regions, and retains a binding affinity to the respiratory syncytial virus fusion protein (preferably the pre-fusion F protein).

In another preferred embodiment, the heavy chain variable region has an amino acid sequence as shown in SEQ ID NO: 1 or 2.

In another preferred embodiment, the heavy chain variable region has an amino acid sequence as shown in SEQ ID NO: 2.

In another preferred embodiment, the heavy chain of the antibody further comprises a heavy chain constant region.

3

In another preferred embodiment, the heavy chain constant region is of human origin.

In another preferred embodiment, the light chain variable region has an amino acid sequence as shown in SEQ ID NO: 4 or 5.

In another preferred embodiment, the light chain variable region has an amino acid sequence as shown in SEQ ID NO: 4.

In another preferred embodiment, the light chain of the antibody further comprises a light chain constant region.

In another preferred embodiment, the light chain constant region is of human origin.

In another preferred embodiment, the antibody is selected from a humanized antibody.

In another preferred embodiment, the antibody is a double-chain antibody or a single-chain antibody.

In another preferred embodiment, the antibody is a monoclonal antibody or a polyclonal antibody.

In another preferred embodiment, the antibody is a partially or fully humanized monoclonal antibody.

In another preferred embodiment, the antibody is in the form of a drug conjugate.

In another preferred embodiment, the antibody has a heavy chain variable region of which the sequence is shown in SEQ ID NO: 1 or 2; and a light chain variable region of which the sequence is shown in SEQ ID NO: 4 or 5.

In another preferred embodiment, the antibody has a heavy chain variable region of which the sequence is shown in SEQ ID NO: 2 and a light chain variable region of which the sequence is shown in SEQ ID NO: 4.

In the second aspect of the present invention, it provides a recombinant protein which comprises:

(i) the antibody according to the first aspect of the present invention; and

(ii) an optional tag sequence to assist expression and/or purification.

In another preferred embodiment, the tag sequence comprises a 6His tag, a GGGS sequence, or a FLAG tag.

In another preferred embodiment, the recombinant protein (or polypeptide) comprises a fusion protein.

In another preferred embodiment, the recombinant protein is a monomer, dimer, or multimer.

In another preferred embodiment, the recombinant protein specifically binds to the respiratory syncytial virus fusion protein, preferably the pre-fusion F protein.

In another preferred embodiment, the recombinant protein is a fusion protein.

In another preferred embodiment, the fusion protein is a monospecific antibody (i.e., a monospecific antibody against RSV fusion protein), a bispecific antibody, or a multispecific antibody (e.g., a trispecific antibody).

In another preferred embodiment, the bispecific antibody or multispecific antibody specifically binds to an additional target antigen (e.g., another viral antigen, such as another antigen of respiratory syncytial virus or an antigen of other viruses) in addition to the RSV fusion protein.

In the third aspect of the present invention, it provides a CAR construct, wherein the scFv fragment of the antigen-binding domain of the CAR construct is a binding region specifically binding to the RSV fusion protein (preferably the pre-fusion F protein), and the scFv comprises the heavy chain variable region and the light chain variable region as described in the first aspect of the present invention.

In the fourth aspect of the present invention, it provides a recombinant immune cell, wherein the immune cell expresses an exogenous CAR construct according to the third aspect of the present invention.

In another preferred embodiment, the immune cell is selected from the group consisting of: NK cells and T cells.

In another preferred embodiment, the immune cell is derived from human or a non-human mammal (such as a mouse).

In the fifth aspect of the present invention, it provides an antibody-drug conjugate, which comprises:

(a) an antibody moiety, which is selected from the group consisting of the antibody according to the first aspect of the present invention, and a combination thereof; and

(b) a coupling moiety coupled to the antibody moiety, which is selected from the group consisting of a detectable label, a drug, a toxin, a cytokine, a radionuclide, an enzyme, and a combination thereof.

In another preferred embodiment, the coupling moiety is selected from the group consisting of a fluorescent or luminescent marker, a radioactive marker, MRI (magnetic resonance imaging) or CT (electronic computer X-ray tomography technique) contrast agent, or an enzyme capable of producing a detectable product, a radionuclide, a biotoxin, a cytokine (such as IL-2, etc.), an antibody, an Fc fragment of an antibody, an scFv fragment of an antibody, a gold nanoparticle/nanorod, a viral particle, a liposome, a magnetic nanoparticle, a prodrug activating enzyme (such as DT-diaphorase (DTD) or biphenyl hydrolase-like protein (BPHL)), chemotherapeutic agent (such as cisplatin) and any form of nanoparticles, and the like.

In another preferred embodiment, the antibody moiety and the coupling moiety are coupled through a chemical bond or a linker.

In the sixth aspect of the present invention, it provides a use of an active ingredient in preparation of a drug, a reagent, a detection plate or a kit, wherein the active ingredient is selected from the group consisting of: the antibody according to the first aspect of the present invention, the recombinant protein according to the second aspect of the present invention, and a combination thereof.

In another preferred embodiment, the reagent, detection plate or kit is used for detecting respiratory syncytial virus.

In another preferred embodiment, the drug is used for treating or preventing infection of respiratory syncytial virus.

In another preferred embodiment, the reagent includes a chip and an immunoparticle coated with the antibody.

In the seventh aspect of the present invention, it provides a pharmaceutical composition, which comprises:

(i) an active ingredient, wherein the active ingredient is selected from the group consisting of: the antibody according to the first aspect of the present invention, the recombinant protein according to the second aspect of the present invention, the immune cell according to the fourth aspect of the present invention, the antibody-drug conjugate according to the fifth aspect of the present invention, and a combination thereof; and

(ii) a pharmaceutically acceptable carrier.

In another preferred embodiment, the pharmaceutical composition is a liquid preparation.

In another preferred embodiment, the pharmaceutical composition is an injection.

In another preferred embodiment, the pharmaceutical composition is used for preventing and/or treating infection of respiratory syncytial virus.

In the eighth aspect of the present invention, it provides a polynucleotide encoding a polypeptide selected from the group consisting of:

(1) the antibody according to the first aspect of the present invention; and

(2) the recombinant protein according to the second aspect of the present invention;

(3) the CAR construct according to the third aspect of the present invention.

In another preferred embodiment, the polynucleotide comprises a sequence as shown in SEQ ID NO: 8 or 9, and/or SEQ ID NO: 11 or 12.

In another preferred embodiment, the polynucleotide comprises a sequence as shown in SEQ ID NO: 9 and/or SEQ ID NO: 11.

In the ninth aspect of the present invention, it provides a vector comprising the polynucleotide according to the eighth aspect of the present invention.

In another preferred embodiment, the vector includes: bacterial plasmid, phage, yeast plasmid, plant cell virus, mammalian cell virus such as adenovirus, retrovirus, or other vectors.

In the tenth aspect of the present invention, it provides a genetically engineered host cell which comprises the vector according to the ninth aspect of the present invention or the genome thereof is integrated with the polynucleotide according to the eighth aspect of the present invention.

In the eleventh aspect of the present invention, it provides a method for detection of respiratory syncytial virus in a sample, wherein the method comprises steps of:

(1) contacting the sample with the antibody according to the first aspect of the present invention;

(2) detecting whether an antigen-antibody complex is formed, wherein the formation of the complex indicates the presence of respiratory syncytial virus in the sample.

In another preferred embodiment, the detection is non-diagnostic and non-therapeutic.

In the twelfth aspect of the present invention, it provides a method for detection of respiratory syncytial virus fusion protein in a sample, wherein the method comprises steps of:

(1) contacting the sample with the antibody according to the first aspect of the present invention;

(2) detecting whether an antigen-antibody complex is formed, wherein the formation of the complex indicates the presence of respiratory syncytial virus fusion protein in the sample.

In another preferred embodiment, the respiratory syncytial virus fusion protein is the respiratory syncytial virus pre-fusion F protein.

In another preferred embodiment, the detection is non-diagnostic and non-therapeutic.

In the thirteenth aspect of the present invention, it provides a detection plate, which comprises a substrate (support plate) and a test strip, and the test strip contains the antibody according to the first aspect of the present invention or the immunoconjugate according to the fifth aspect of the present invention.

In the fourteenth aspect of the present invention, it provides a kit, which comprises:

(1) a first container, which contains the antibody according to the first aspect of the present invention; and/or
(2) a second container, which contains a secondary antibody against the antibody according to the first aspect of the present invention;

or, the kit comprises the detection plate according to the thirteenth aspect of the present invention.

In the fifteenth aspect of the present invention, it provides a method for preparing a recombinant polypeptide, which comprises:

(a) culturing the host cell according to the tenth aspect of the present invention under conditions suitable for expression;
(b) isolating a recombinant polypeptide from the culture, wherein the recombinant polypeptide is the antibody according to the first aspect of the present invention or the recombinant protein according to the second aspect of the present invention.

In the sixteenth aspect of the present invention, it provides a method for treating infection of respiratory syncytial virus, which comprises: administering the antibody according to the first aspect of the present invention, an antibody-drug conjugate of the antibody, or a CAR-T cell expressing the antibody, or a combination thereof to a subject in need thereof.

It should be understood that, within the scope of the present invention, the technical features specifically described above and below (such as the Examples) can be combined with each other, thereby constituting a new or preferred technical solution which needs not be described one by one.

## DESCRIPTION OF DRAWINGS

The following drawings are used to illustrate specific embodiments of the present invention, and are not intended to limit the scope of the invention as defined by the claims.

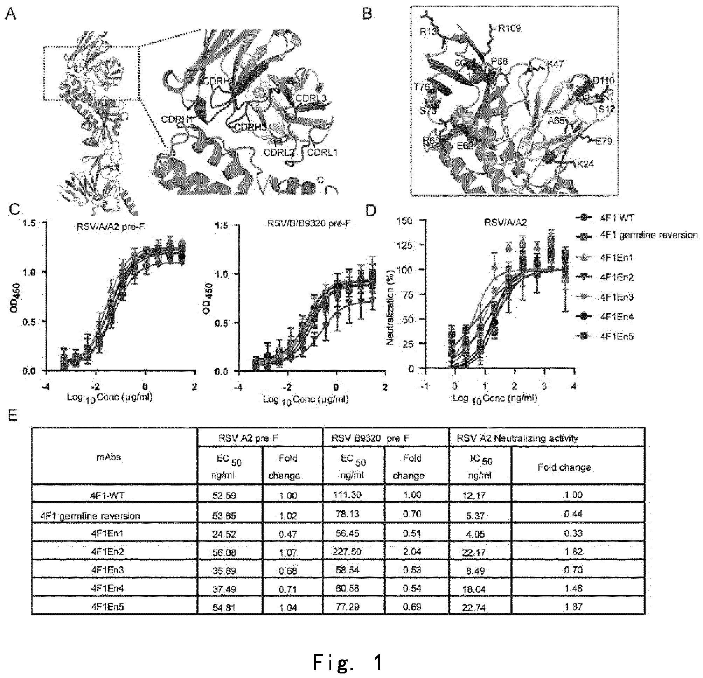

Figure 1 shows using computer-aided design to optimize and enhance the binding and neutralizing activities of 4F1 antibodies. (A) The mock structure of 4F1 and A2/pre-F protein molecules docking. Cyan indicates the 4F1 heavy chain; light cyan indicates the 4F1 light chain; green indicates pre-F; rose red indicates the three variable regions of the heavy chain, CDRH1, CDRH2, and CDRH3; and orange indicates the three variable regions of the light chain, CDRL1, CDRL2, and CDRL3. The structure of the pre-F protein was derived from PDB:4JHW. (B) Reversion of the somatic mutation sites of 4F1 antibody back to the amino acid corresponding to the germline on the basis of structural analysis. (C) The binding activity of structural optimized 4F1 mutants with RSV/A/A2 and RSV/B/B9320 pre-F proteins validated by ELISA. (D) Neutralization capacity of different 4F1 mutants against RSV/A/A2. RSV viruses were incubated with different concentrations of antibodies for 1 h and then added to trypsin-digested HEp2 cells. After 24 h of incubation, the supernatant was discarded, and the cells were fixed in 80% acetone for 30 min, and the viral titers were detected by ELISA with anti-F and G antibodies. The IC50 was calculated. (E) Statistical analysis of the binding of different 4F1 mutants to pre-F proteins and the comparison of neutralization activity against RSV/A/A2. EC50 is the concentration of antibody that produces 50% maximal response. The fold change in binding was calculated as follows: EC50 of 4F1 mutant/EC50 of 4F1 wild type.

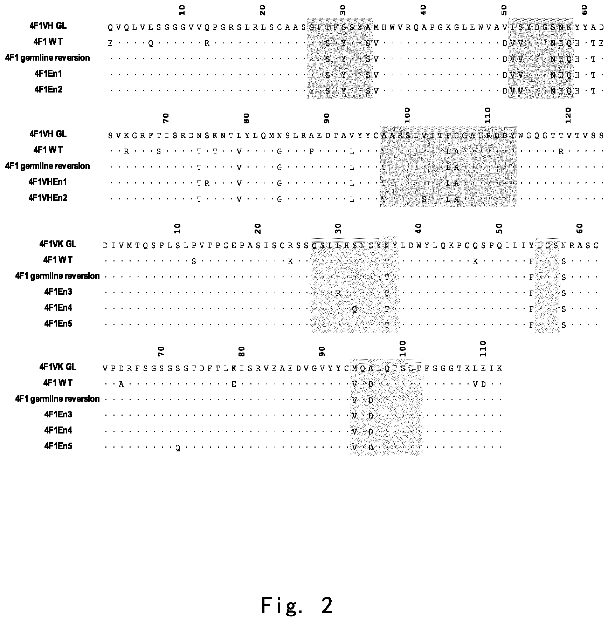

Figure 2 shows a sequence alignment of the optimized 4F1 antibodies with the 4F1 wild type as well as the germline antibody. "·" indicates identical amino acids. Gray shading indicates the three CDR regions of the heavy and light chains.

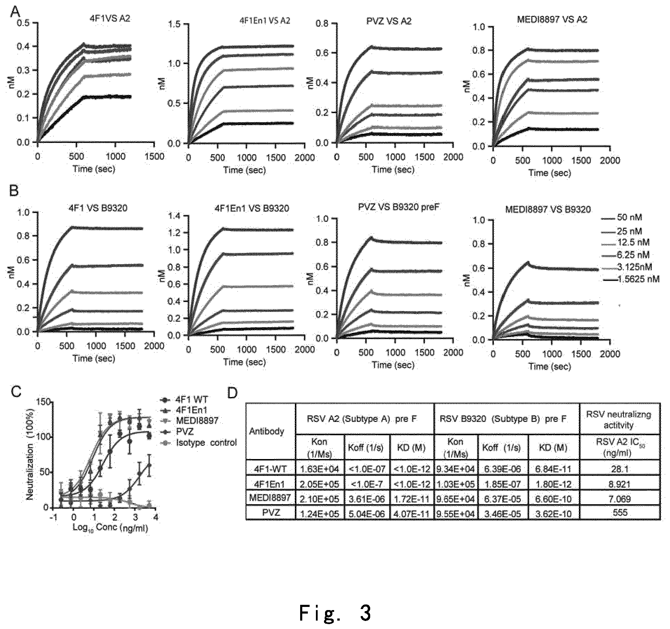

Figure 3 shows that the optimized 4F1En1 antibody has higher affinity and neutralizing activity. (A) Affinity of different antibodies to RSV/A/A2 pre-F protein. (B) Affinity of different antibodies to RSV/B/B9320 pre-F protein. Affinity assay was performed using the ForteBio Octet® RED96 Interaction Analyzer. The AHC sensor was used according to the following procedure: baseline 120s-loading Ab 300s-baseline 600s-association 1200s-disassociation 900s-regenaration 5s-baseline 5s-regenaration 5s-baseline 5s-regenaration 5s-baseline 5s. Antibody concentration for loading was 5ug/ml, and six concentrations were set for pre-F antigen: 50 nM-25 nM-12.5 nM-6.25 nM-3.125 nM-1.5625 nM. Kd values were calculated. (C) Neutralization ability of different antibodies against RSV-A2 strain. (D) Comparative analysis of affinity and neutralizing activity of 4F1En1 with the first-generation and the second-generation RSV neutralizing antibodies, Palivizumab (PVZ) and MEDI8897.

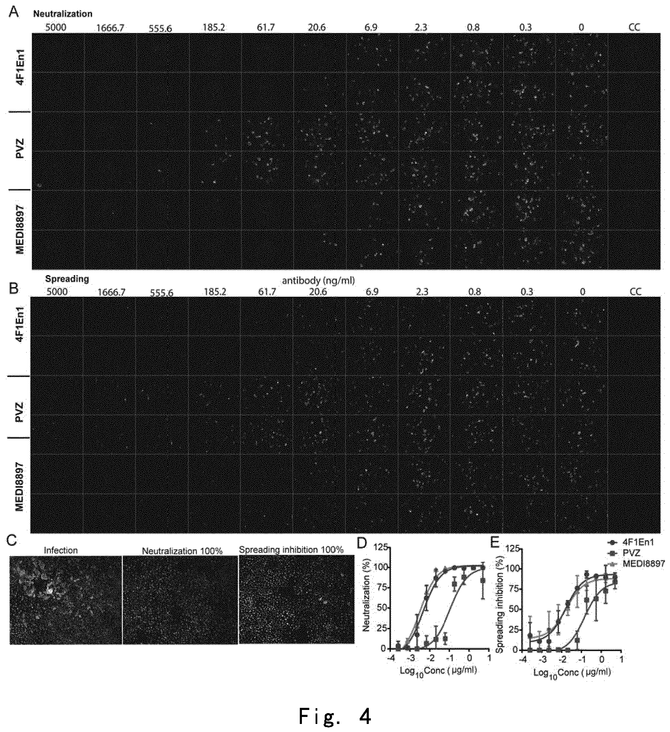

Figure 4 shows the ability of 4F1En1 for inhibiting infection and spread of RSV verified by a high content screening system. (A) Ability for neutralizing RSV virus. Series diluted antibodies were incubated with 500 pfu RSV/A2 for 1 hour and added to HEp-2 cells. After 3 days, virus-infected cells were stained with biotinylated anti-F and G monoclonal antibodies. The inhibition of viral infection was detected with FITC-labeled streptavidin. Photographs were taken by Opera high content screening system. (B) Verification of antibody inhibition of RSV spread. Series diluted antibodies were added to HEp-2 cells pre-infected with RSV/A2 for 24 hours, and the inhibition of viral infection was determined on day 3 as described above. For A and B, images were taken at $2\times$ magnification. (C) 100% protective effect of neutralization and diffusion inhibition at $10\times$ magnification. Left figure shows the formation of larger syncytia without antibody addition; center figure shows the uninfected cells in the neutralization assay; right figure shows the infected single-cells in the diffusion inhibition assay. Cell nuclei were stained by DAPI for comparison. (D-E) The neutralization and diffusion inhibition effects of antibodies were quantified by using statistical analyses based on $10\times$ magnification. PVZ and MEDI8897 were used as controls. These data represent at least two independent experiments.

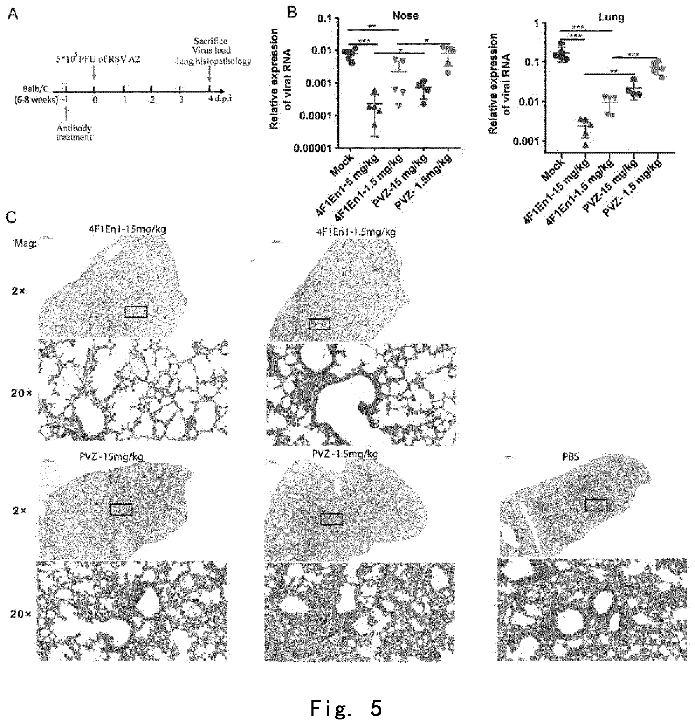

Figure 5 shows the comparison of the preventing effect of 4F1 En1 and Palivizumab (PVZ) against RSV in mice. (A) Schematic of preventive administration in mice. 6-8-week-old BALB/c mice (n=4-6) were injected with 15 mg/kg and 1.5 mg/kg of 4F1 antibody and PVZ or diluent as a control 24 hours prior to intranasal (i.n.) infection with $5*10^5$ p.f.u. RSV a/A2. (B) Mice were euthanized on day 4 (d.p.i.). Their nasal turbinates and lungs were isolated and homogenized to extract viral RNA. Samples were subjected to qPCR using $\beta$-actin- and HRSV NP-specific primers. P-values were calculated through unpaired Student t-test. * $P<0.05$, ** $P<0.01$, *** $P<0.001$, *** $P<0.0001$, NS, no significance. (C) Hematoxylin-eosin staining of mouse lung sections was performed at 4 dpi. Images were shown at $2\times$ magnification (top; scale bar, 500 $\mu$m) and $20\times$ magnification (bottom; scale bar, 50 $\mu$m).

## DETAILED DESCRIPTION

After extensive and intensive research, the present inventors have developed the design and use of a new fully human antibody for neutralizing respiratory syncytial virus. The present inventors obtained a novel antibody with significantly improved properties by performing germline gene reverse mutation and affinity enhancement on fully human monoclonal antibody 4F1 against the respiratory syncytial virus fusion protein through extensive screening combined with site-directed mutagenesis. The modified novel antibody has an amino acid sequence with relatively less somatic mutation sites, while its affinity constants for F proteins of different subtypes of RSV reach picomolar level and are superior to the two clinically available antibodies. *In vitro* and *in vivo* experiments confirm that the antibodies of the present invention are effective in preventing RSV infection and inhibiting the spread of RSV after infection. On this basis, the present invention has been completed.

### Terms

To make it easier to understand the present invention, certain technical and scientific terms are specifically defined below. Unless otherwise clearly defined herein, all other technical and scientific terms used herein have meanings commonly understood by those skilled in the art in the field to which the present invention belongs. Before describing the present invention, it should be understood that the present invention is not limited to the specific methods and experimental conditions described, as such methods and conditions can be changed. It should also be understood that the terms used herein are only intended to describe the specific embodiments and are not intended to be restrictive. The scope of the present invention will be limited only by the accompanying claims.

Unless otherwise defined, all technical and scientific terms used herein have the same meanings as those commonly understood by those skilled in the art to which the present invention belongs. As used herein, when used in reference to specific enumerated values, the term "about" means that the value may vary by no more than 1% from the enumerated value. For example, as used herein, "about 100" includes all values between 99 and 101 (e.g., 99.1, 99.2, 99.3, 99.4, etc.).

The three-letter codes and one-letter codes of amino acids used in the present invention are as described in J. biol. chem, 243, p3558 (1968).

As used herein, the term "treatment" refers to the administration of an internal or external therapeutic agent, including the antibody targeting the respiratory syncytial virus fusion protein (preferably the pre-fusion F protein) of the present invention and the composition thereof, to a patient who has one or more disease symptoms, wherein the therapeutic agent is known to have a therapeutic effect on these symptoms. Usually, the therapeutic agent is administered to a patient with an amount effective to alleviate one or more disease symptoms (a therapeutically effective amount).

As used herein, the term "optional" or "optionally" means that the event or situation described thereafter may occur, but not necessarily. For example, "optionally comprises 1-3 antibody heavy chain variable regions" means that the antibody heavy chain variable region of a specific sequence may be comprised but does not have to be comprised, and the number may be 1, 2 or 3.

The "sequence identity" described in the present invention means the degree of identity between two nucleic acid sequences or two amino acid sequences when optimally aligned and compared, with appropriate mutations such as substitutions, insertions or deletions. The sequence identity between the sequence and its identical sequence described in the present invention may be at least 85%, 90% or 95%, preferably at least 95%. Non-limiting examples include 85%, 86%, 87%, 88%, 89%, 90%, 91%, 92%, 93%, 94%, 95%, 96%, 97%, 98%, 99%, 100%.

**Respiratory Syncytial Virus (RSV)**

RSV belongs to *Paramyxoviridae* family, *Pneumovirus* genus, and it is a negative-stranded RNA virus consisting of 15222 nucleotides. RSV is an enveloped virus, and its genome encodes a total of 11 proteins, including fusion protein F, attachment protein G, small hydrophobic protein SH, matrix protein M, nucleoprotein N, phosphoprotein P, large polymerase protein L, small matrix protein M2, and nonstructural proteins NS1 and NS2, wherein the fusion protein F, attachment protein G and small hydrophobic protein SH are located on the surface of the virus. RSV has two major surface glycoproteins, G and F. The G protein mediates the binding of virus to the surface of cell and also mimics the action of the chemokine CX3C and interacts with its receptor to enhance the inflammatory response after RSV infection. The F protein mediates the fusion of the virus with the cell membrane and also promotes the fusion between the membrane of the infected cell and the surrounding cells to form syncytia. Serologically, there are two distinct antigenic groups or subtypes of RSV, A and B, which are distinguished primarily by differences in G proteins. Most RSV proteins are highly conserved between these two subgroups, with fusion F proteins showing 91% amino acid similarity and G proteins having only 53% homology between subtypes A and B. The F protein is highly conserved, and the neutralizing antibody induced thereby can inhibit viral infection of both A and B subtypes, which is important in the development of anti-RSV monoclonal antibody drugs and vaccines. F protein belongs to type I transmembrane proteins. F protein is formed by first synthesis of the precursor protein F0. F0 is trimerized in the endoplasmic reticulum and processed by cytosolic furin-like protease at two conserved sites to produce F1, F2, and Pep27 polypeptides. Finally, two fragments of F1 and F2 linked by disulfide bonds are formed, and the F2-F1 heterodimer forms a trimer to assemble the mature F protein. The F1 subunit contains a heptapeptide repeat region A (HRA), functional domains I/II, s heptapeptide repeat region B (HRB), a transmembrane protein (TM), and a cytoplasmic region (CP), while the F2 subunit consists of a heptapeptide repeat region C (HRC). The F1 hydrophobic N-terminus (137-155) plays an important role in mediating fusion. The membrane fusion process mediated by the F protein is also a process where its structure transforms from the pre-fusion state to the fusion state. It adopts a substable pre-fusion conformation. When the G protein binds to the receptor on the target cell membrane, it triggers allosterism of the pre-fusion F protein to a stable post-fusion form. Due to its critical role in RSV invasion and high conservation, the RSV F protein is a target for neutralizing antibodies and a major antigen for vaccine development. Numerous studies have demonstrated that the recognition sites of neutralizing antibody against the F protein are predominantly on the pre-fusion F protein, and that recombinant proteins designed on the basis of the pre-fusion F are significantly more immunogenic and protective than vaccines designed on the basis of the post-fusion F protein.

As used herein, the terms "pre-fusion F protein" and "preF protein" can be used interchangeably to refer to the pre-fusion form of respiratory syncytial virus (RSV) fusion protein F.

In order to further obtain novel RSV antibody drugs with better efficacy and advanced physicochemical properties, we modified and optimized a broad-spectrum neutralizing antibody 4F1 against RSV F protein by using site-directed mutagenesis technology and computer-aided design. On one hand, the optimized novel antibody provides a better choice for broad-spectrum neutralizing antibody therapy and application. On the other hand, through the implementation of this project, a clearer understanding of the epitopes recognized by the antibody and the mechanism of viral neutralization is obtained, which provides new targets for drug design.

**Antibody**

The term "antibody" or "immunoglobulin" as used herein refers to a heterotetrameric glycoprotein having the same structural feature of about 150,000 daltons consisting of two identical light chains (L) and two identical heavy chains (H). Each light chain is linked to a heavy chain by a covalent disulfide bond, and the numbers of disulfide bonds between the heavy chains of different immunoglobulin isoforms are different. Each heavy and light chain also has regularly spaced intrachain disulfide bonds. One end of each heavy chain has a variable region (VH) followed by a plurality of constant regions. There is a variable region (VL) at one end of each chain and a constant region at the other end; the constant region of the light chain corresponds to the first constant region of the heavy chain; the variable region of the light chain corresponds to the variable region of the heavy chain. An interface is formed between the variable regions of the light and heavy chains by particular amino acid residues.

As used herein, the term "variable" means that some certain portions of the variable region of an antibody differ in sequence and contribute to the binding and specificity of each particular antibody to its particular antigen. However, the variability is not evenly distributed throughout the antibody variable region. It is concentrated in three regions in the light and heavy chain variable regions called complementarity determining regions (CDRs) or hypervariable regions. The more conserved portions of the variable regions are referred as framework regions (FRs). The variable regions of the natural heavy and light chains each comprises four FR regions, which are in a substantially β-folded configuration, and are linked by three CDRs that form the linker ring and, in some cases, form a partial β-folded structure. The CDRs in each chain stand close together through FR regions and form the antigen-binding site of the antibody together with the CDRs of the other chain (see Kabat et al., NIH Publ. No. 91-3242, Vol. I, 647-669 (1991)). Constant regions are not directly involved in the binding of the antibodies to the antigens, but they exhibit different effector functions, such as antibody-dependent cellular cytotoxicity involved in antibodies.

The "light chain" of vertebrate antibodies (immunoglobulins) can be classified into one of two distinct categories (called kappa and lambda) based on the amino acid sequence of their constant region. Immunoglobulins can be classified into different types based on the amino acid sequence of their heavy chain constant region. There are mainly 5 types of immunoglobulins: IgA, IgD, IgE, IgG, and IgM, some of which can be further divided into subclasses (isotypes), such as IgG1, IgG2, IgG3, IgG4, IgA, and IgA2. The heavy chain constant regions corresponding to different types of immunoglobulins are called $\alpha$, $\delta$, $\epsilon$, $\gamma$, and $\mu$, respectively. The subunit structures and three-dimensional configurations of different types of immunoglobulins are well-known to those in this field.

As used herein, the term "monoclonal antibody (mAb)" refers to an antibody obtained from a substantially homogeneous population, i.e., the individual antibodies contained in the population are the same, except for a few naturally occurring mutations that may be present. A monoclonal antibody is highly specific for a single antigenic site. Moreover, unlike conventional polyclonal antibody preparations (usually with different antibodies directed against different determinants), each monoclonal antibody is directed against a single determinant on the antigen. In addition to their specificity, the advantage of monoclonal antibodies is that they can be synthesized by hybridoma culture and will not be contaminated by other immunoglobulins. The modifier "monoclonal" indicates the characteristics of antibodies, which are obtained from a substantially uniform antibody population, which should not be interpreted as requiring any special method to produce antibodies.

The present invention also includes a monoclonal antibody with corresponding amino acid sequences of the monoclonal antibody against respiratory syncytial virus fusion protein (preferably the pre-fusion F protein), a monoclonal antibody with variable region chains of a monoclonal antibody against respiratory syncytial virus fusion protein (preferably the pre-fusion F protein), as well as other proteins or protein conjugates and fusion expression products with these chains. Specifically, the present invention includes any protein or protein conjugate and fusion expression product (i.e., immunoconjugate and fusion expression product) having a heavy chain and a light chain containing hypervariable regions, as long as the hypervariable region is the same as or has at least 90% homology with the hypervariable regions of the heavy chain and light chain of the antibody of the present invention, preferably at least 95% homology.

As known to those skilled in the art, an immunoconjugate or a fusion expression product includes: a conjugate formed by a drug, a toxin, a cytokine, a radionuclide, an enzyme and other diagnostic or therapeutic molecules combining with the monoclonal antibody or a fragment thereof against respiratory syncytial virus fusion protein of the present invention. The present invention also includes a cell surface marker or antigen that binds to the monoclonal antibody or a fragment thereof against respiratory syncytial virus fusion protein.

The term "antigen-binding fragment of an antibody" (or abbreviated "antibody fragment") refers to one or more fragments of an antibody that retain the ability to specifically bind to an antigen. It has been shown that fragments of a full-length antibody can be used to perform the antigen binding function of the antibody. Examples of binding fragments included in the term "antigen-binding fragment of an antibody" include (i) an Fab fragment, a monovalent fragment composed of VL, VH, CL, and CH1 domains; (ii) an F(ab')$_2$ fragment, a divalent fragment containing two Fab fragments connected by a disulfide bridge between hinge regions; (iii) an Fd fragment composed of VH and CH1 domains; (iv) an Fv fragment composed of the VH and VL domains of the single arm of an antibody. An Fv antibody comprises a heavy chain variable region and a light chain variable region, but does not comprise a constant region, and it is the smallest antibody fragment with all antigen binding sites. Generally, an Fv antibody also comprises a polypeptide linker between the VH and VL domain, and can form the structure required for antigen binding.

The present invention includes not only a complete monoclonal antibody, but also an antibody fragment with immune activity, such as an Fab or (Fab')$_2$ fragment, an antibody heavy chain, and an antibody light chain.

The term "epitope" or "antigenic determinant" refers to the site on an antigen where the immunoglobulin or the antibody specifically binds. Epitope usually comprises at least 3, 4, 5, 6, 7, 8, 9, 10, 11, 12, 13, 14 or 15 consecutive or non-consecutive amino acids in a unique spatial conformation.

The terms "specific binding", "selective binding", "selectively bind", and "specifically bind", refer to the binding of an antibody to an epitope on a predetermined antigen. Usually, an antibody has an affinity (KD) that is approximately less than $10^{-7}$M, such as approximately less than $10^{-8}$ M, $10^{-9}$ M or $10^{-10}$ M or less.

As used herein, the term "antigen determinant" refers to a discrete three-dimensional site on an antigen that is recognized by the antibody or antigen-binding fragment of the present invention.

The present invention includes not only intact antibodies, but also immunologically active fragments of antibody fragments or fusion proteins formed by antibodies and other sequences. Therefore, the present invention also includes fragments, derivatives and analogs of the antibodies.

In the present invention, antibodies include murine, chimeric, humanized, or fully human antibodies prepared by techniques well known to those skilled in the art. Recombinant antibodies, such as chimeric and humanized monoclonal antibodies, including human and non-human parts, can be prepared using DNA recombinant techniques well known in the art. The term "murine antibody" in the present invention refers to a monoclonal antibody prepared based on knowledge and skills in the art that targets the respiratory syncytial virus fusion protein. The term "chimeric antibody" is an antibody formed by fusing the variable region of a mouse antibody with the constant region of a human antibody, which can reduce the immune response induced by the mouse antibody. The term "humanized antibody", also known as CDR-grafted antibody, refers to the antibody produced by transplantation of mouse CDR sequences into the framework regions of human antibody variable regions (i.e., different types of human germline antibody framework sequences). Humanized antibodies can overcome the heterogeneous reaction induced by chimeric antibodies that carry a large amount of mouse protein components. Such framework sequences can be obtained from public DNA databases or published references that include germline antibody gene sequences. In order to avoid the decrease in activity resulting from the decrease of immunogenicity, the human antibody variable region framework sequence can be subjected to minimal reverse mutations or back mutations to maintain activity.

In the present invention, the antibody may be monospecific, bispecific, trispecific, or more multispecific.

As used herein, the terms "heavy chain variable region" and "VH" can be used interchangeably.

As used herein, the terms "variable region" and "complementarity determining region (CDR)" can be used interchangeably.

The term "CDR" refers to one of the 6 hypervariable regions within the variable domain of an antibody that mainly contributes to antigen binding. One of the most commonly used definitions of the 6 CDRs is provided by Kabat E.A et al., (1991) Sequences of proteins of immunological interest. NIH Publication 91-3242.

In a preferred embodiment of the present invention, the amino acid sequence of the heavy chain variable region of the antibody is shown in SEQ ID NO: 1, 2 or 3.

In another preferred embodiment, the heavy chain variable region has an amino acid sequence as shown in SEQ ID NO: 2.

In another preferred embodiment, the nucleic acid coding sequence of the heavy chain variable region is shown in SEQ ID NO: 9.

In a preferred embodiment of the present invention, the heavy chain of the antibody comprises the aforementioned heavy chain variable region and heavy chain constant region, which may be of mouse origin or human origin.

As used herein, the terms "light chain variable region" and "VL" can be used interchangeably.

In a preferred embodiment of the present invention, the amino acid sequence of the light chain variable region of the antibody is shown in SEQ ID NO: 4, 5, 6 or 7.

In another preferred embodiment, the amino acid sequence of the light chain variable region is shown in SEQ ID NO: 4.

In another preferred embodiment, the nucleic acid coding sequences of the light chain variable region is shown in SEQ ID NO: 11.

In a preferred embodiment of the present invention, the light chain of the antibody comprises the aforementioned light chain variable region and light chain constant region, which may be of mouse origin or human origin.

The function of the antibody of the present invention is determined by the specific gene sequences of the variable regions of the light and heavy chains of the antibody. The antibody of the present invention has low immunogenicity and can bind to the pre-fusion F protein of RSV type A and B viruses with high affinity, which can prevent respiratory syncytial virus from infecting susceptible cells. Using the variable region gene or complementarity determining region (CDR) gene of the antibody, different forms of genetically engineered antibodies can be modified and produced in any expression system using prokaryotic and eukaryotic cells.

In the present invention, the terms "the antibody of the present invention", "the protein of the present invention", or "the polypeptide of the present invention" can be used interchangeably and all refer to an antibody specifically binding to respiratory syncytial virus fusion protein (preferably the pre-fusion F protein), e.g., a protein or polypeptide with a heavy chain variable region (such as the amino acid sequence encoded by the nucleotide sequence as shown in SEQ ID NO.: 9) and/or a light chain variable region (such as the amino acid sequence encoded by the nucleotide sequence as shown in SEQ ID NO.: 11). They can contain or do not contain starting methionine.

In another preferred embodiment, the antibody is a human monoclonal antibody against respiratory syncytial virus fusion protein (preferably the pre-fusion F protein), and its heavy chain constant region and/or light chain constant region may be humanized heavy chain constant region or light chain constant region. More preferably, the humanized heavy chain constant region or light chain constant region is the heavy chain constant region or light chain constant region of human IgG1, IgG2, etc.

In general, the antigen-binding properties of an antibody can be described by three specific regions located in the heavy chain variable region, referring as variable regions (CDRs), and separated into four framework regions (FRs). The sequences of four FRs amino acids are relatively conservative and do not directly participate in the binding reaction. A cyclic structure is formed by these CDRs which are close to each other in the spatial structure by the β-folds formed by the FRs between them, and the CDRs on the heavy chains and the CDRs on the corresponding light chains constitute the antigen-binding sites of the antibody. The amino acid sequence of the same type of antibody can be used to determine which amino acids have constituted the FR or CDR region.

The variable regions of the heavy chain and/or the light chain of the antibody of the present invention are of particular interest because at least part of them involves binding antigens. Therefore, the present invention includes molecules with light and heavy chain variable regions of monoclonal antibodies with CDRs, as long as their CDR has more than 90% (preferably more than 95%, most preferably more than 98%) homology with the CDR identified here.

The present invention includes not only intact monoclonal antibodies, but also fragments of immunologically active antibodies or fusion proteins formed by antibodies with other sequences. Thus, the present invention also includes fragments, derivatives and analogs of the antibody. For example, modification has been performed on the Fc fragment based on the antibody of the present invention. Three mutation sites M252Y /S254T /T256E are introduced into the CH2 region in order to prolong the half-life of the antibody.

As used herein, the terms "fragment", "derivative" and "analog" refer to the polypeptides basically maintaining the same biological function or activity of the antibody of the present invention. The polypeptide fragments, derivatives or analogs of the present invention may be (i) a polypeptide with one or more conservative or non-conservative amino acid residues (preferably the conservative amino acid residues) being substituted, while such substituted amino acid residues may or may not be encoded by genetic code, or (ii) a polypeptide having substituted group(s) in one or more amino acid residues, or (iii) a polypeptide formed by fusion of the matured polypeptide with another compound (such as the compound that prolongs the half-life of the polypeptide, such as polyethylene glycol), or (iv) a polypeptide formed with additional amino acid sequence fused to said polypeptide sequence (such as, leader sequence, secretion sequence, or a sequence or a protein sequence used to purify the polypeptide, or a fusion protein formed with 6His tag). According to the subject application, these fragments, derivatives and analogs are within the scope commonly known by the skilled person.

The antibody of the present invention refers to a polypeptide having respiratory syncytial virus fusion protein (preferably the pre-fusion F protein) binding activity and comprising the above-mentioned CDR regions. The term also includes variant forms of polypeptides comprising the CDR regions described above that have the same function as the antibody of the present invention. These variant forms include, but are not limited to, deletion, insertion and/or substitution of one or more amino acids (typically 1-50, preferably 1-30, more preferably 1-20, most preferably 1-10), and the addition of one or more amino acids (typically at most 20, preferably at most 10, more preferably at most 5) at the C-terminus and/or N-terminus. For example, in the art, the protein's functions are usually unchanged when an amino acid is substituted by a similar or analogous one. Also, for example, the addition of one or more amino acids at the C-terminus and/or the N-terminus usually will not alter the function of the protein. The term also includes active fragments and active derivatives of the antibody of the present invention.

The variant forms of the antibody include homologous sequences, conserved variants, allelic variants, natural mutants, induced mutants, proteins encoded by a DNA capable of hybridizing to the coding DNA of the antibody of the present invention under high or low stringency conditions, and a polypeptide or protein obtained from the antiserum of the antibody of the present invention.

The present invention also provides other polypeptides, such as fusion proteins containing human antibodies or fragments thereof. In addition to the almost full-length polypeptide, the present invention also includes fragments of the human antibody of the present invention. Typically, the fragment has at least about 50 contiguous amino acids, preferably at least about 60 contiguous amino acids, more preferably at least about 80 contiguous amino acids, and most preferably at least about 100 contiguous amino acids of the antibody of the present invention.

In the present invention, "the conservative variant of the antibody of the present invention" refers to a polypeptide that comprises at most 10, preferably at most 8, more preferably at most 5, most preferably at most 3 amino acids substituted by amino acids with the same or similar properties compared with the amino acid sequence of the antibody of the present invention. These conservatively variant polypeptides are preferably produced by amino acid substitution according to Table A.

Table A

| Initial residue | Representative substitution | Preferred substitution |
|---|---|---|
| Ala (A) | Val; Leu; Ile | Val |
| Arg (R) | Lys; Gln; Asn | Lys |
| Asn (N) | Gln; His; Lvs; Arg | Gin |

(continued)

| Initial residue | Representative substitution | Preferred substitution |
|---|---|---|
| Asp (D) | Glu | Glu |
| Cys (C) | Ser | Ser |
| Gln (Q) | Asn | Asn |
| Glu (E) | Asp | Asp |
| Gly (G) | Pro; Ala | Ala |
| His (H) | Asn; Gln; Lys; Arg | Arg |
| Ile (I) | Leu; Val; Met; Ala; Phe | Leu |
| Leu (L) | Ile; Val; Met; Ala; Phe | Ile |
| Lys (K) | Arg; Gln; Asn | Arg |
| Met (M) | Leu; Phe; Ile | Leu |
| Phe (F) | Leu; Val; Ile; Ala; Tyr | Leu |
| Pro (P) | Ala | Ala |
| Ser (S) | Thr | Thr |
| Thr (T) | Ser | Ser |
| Trp (W) | Tyr; Phe | Tyr |
| Tvr (Y) | Trp; Phe; Thr; Ser | Phe |
| Val (V) | Ile; Leu; Met; Phe; Ala | Leu |

The present invention also provides a polynucleotide molecule encoding the antibody or a fragment thereof or a fusion protein thereof. The polynucleotides of the present invention can be in a form of DNA or RNA. DNA forms include cDNA, genomic DNA, or synthetic DNA. DNA can be single-stranded or double-stranded. DNA can be the coding strand or the non-coding strand. The coding region sequence encoding mature polypeptide may be the same as or a degenerate variant of the coding region sequence shown in SEQ ID NO.: 4 or 8. As used herein, "degenerate variant" in the present invention refers to a nucleic acid sequence encoding the same amino acid sequence as the polypeptide of the present invention, but different from the coding region sequence shown in SEQ ID NO.: 8, 9, 10, 11, 12, 13 or 14.

The polynucleotides encoding the mature polypeptides of the present invention comprise coding sequences encoding only the mature polypeptide; coding sequences of the mature polypeptide and various additional coding sequences; coding sequences (and optionally additional coding sequences) of the mature polypeptide, and non-coding sequences.

The term "polynucleotide encoding a polypeptide" may include a polynucleotide that encodes the polypeptide, or a polynucleotide that also includes additional coding and/or non-coding sequences.

A polynucleotide that hybridizes with the aforementioned sequence and has at least 50%, preferably at least 70%, and more preferably at least 80% identity between the two sequences, is also involved in the present invention. A polynucleotide that can hybridize with the polynucleotide of the present invention under strict conditions is particularly related to the present invention. In the present invention, "strict conditions" refer to: (1) hybridization and elution at lower ionic strength and higher temperature, such as 0.2×SSC, 0.1% SDS, 60°C; or (2) a denaturant is added during hybridization, such as 50% (v/v) formamide, 0.1% calf serum/0.1% Ficoll, 42°C, etc.; or (3) the hybridization occurs only when the identity between two sequences is at least more than 90%, preferably more than 95%. Furthermore, the polypeptide encoded by the hybridizable polynucleotide has the same biological function and activity as the mature polypeptide shown in SEQ ID NO.: 1, 2, 3, 4, 5, 6 and/or 7.

The whole length of the nucleotide sequence or the fragment thereof of the antibody of the present invention can be obtained via PCR amplification, recombinant method or artificial synthesis. One feasible method is to synthesize relevant sequences by artificial method, especially when the fragment is short in length. Usually, several small fragments are synthesized first, and then are linked together to obtain a fragment with a long sequence. In addition, the sequence coding the heavy chain and the expression label (e.g., 6His) can be fused together to form a fusion protein.

Once a relevant sequence is obtained, recombination methods can be used to obtain the relevant sequence in large quantities. This is usually carried out by cloning the sequence into a vector, transforming a cell with the vector, and then separating the relevant sequence from the proliferated host cell by conventional methods. The biomolecules (nucleic acids, proteins, etc.) involved in the present invention include biomolecules that exist in an isolated form.

At present, DNA sequences encoding the protein of the invention (or fragments thereof, or derivatives thereof) can be completely obtained by chemical synthesis. The DNA sequence can then be introduced into a variety of existing DNA molecules (or vectors) and cells known in the art. In addition, mutations can also be introduced into the protein sequences of the present invention by chemical synthesis.

The present invention further relates to a vector comprising said suitable DNA sequence and a suitable promoter or a control sequence. These vectors can be used to transform suitable host cells to enable them to express protein.

The host cell can be a prokaryotic cell, such as a bacterial cell; or a lower eukaryotic cell, such as a yeast cell; or a higher eukaryotic cell, such as a mammalian cell. Representative examples are: *Escherichia coli, streptomactinus,* the bacterial cell of *salmonella typhimurium;* a fungal cell such as a yeast; an insect cell of Drosophila S2 or SF9; an animal cell of CHO, COS7, 293 cells, etc.

Transformation of a host cell with a recombinant DNA can be carried out by conventional techniques well known to those skilled in the art. When the host is a prokaryotic organism such as Escherichia coli, competent cells that can absorb DNA can be harvested after the exponential growth phase then treated with the CaCh method, and the steps used are well known in the art. Another method is to use $MgCl_2$. If necessary, the transformation can also be carried out by electroporation. When the host is a eukaryote, the following DNA transfection methods can be selected: calcium phosphate co-precipitation method, conventional mechanical methods such as microinjection, electroporation, liposome packaging, etc.

The obtained transformants can be cultured by conventional methods to express the polypeptide encoded by the gene of the present invention. Depending on the host cell used, the medium used during the culture can be selected from various conventional mediums. The culture is carried out under conditions suitable for the growth of the host cell. When the host cell has grown to an appropriate cell density, a suitable method (such as temperature conversion or chemical induction) is used to induce the selected promoter, and the cell is cultured for another period of time.

The recombinant polypeptide in the above method can be expressed intracellularly or on the cell membrane, or be secreted out of the cell. If desired, recombinant proteins can be isolated and purified by various separation methods using their physical, chemical and other properties. These methods are well known to those skilled in the art. Examples of such methods include, but are not limited to, conventional renaturation treatments, treatment with a protein precipitant (salting-out method), centrifugation, osmosis cell disruption, super-treatment, ultracentrifugation, molecular sieve chromatography (gel filtration), adsorption chromatography, ion-exchange chromatography, high performance liquid chromatography (HPLC) and various other liquid chromatography techniques and combinations of these methods.

The antibody of the present invention can be used alone, or can be combined or coupled with a detectable label (for diagnostic purposes), a therapeutic agent, a PK (protein kinase) modifying moiety, or any combination of these substances.

Detectable labels for diagnostic purposes include, but are not limited to: fluorescent or luminescent labels, radioactive labels, MRI (magnetic resonance imaging) or CT (electronic computer X-ray tomography technique) contrast agents, or enzymes capable of producing a detectable product.

Coupled therapeutic agents include but are not limited to: insulin, IL-2, interferon, calcitonin, GHRH peptide, intestinal peptide analog, albumin, antibody fragment, cytokine, and hormone.

In addition, therapeutic agents that can combined or coupled with the antibody of the present invention include but are not limited to: 1. radionuclides; 2. biotoxin; 3. cytokines such as IL-2; 4. gold nanoparticles/nanorods; 5. virus particles; 6. liposomes; 7. magnetic nanoparticles; 8. prodrug-activating enzymes; 10. chemotherapeutic agents (e.g., cisplatin), or nanoparticles in any form, etc.

The present invention further provides a composition. In the preferred embodiments, the composition is a pharmaceutical composition comprising the antibody, or an active fragment thereof or a fusion protein thereof, and a pharmaceutically acceptable carrier. In general, these substances may be formulated in a non-toxic, inert and pharmaceutically acceptable aqueous carrier medium, wherein the pH is generally about 5-8, preferably, pH is about 6-8, though the pH value may be varied depending on the nature of the substances to be formulated and the condition to be treated. The formulated pharmaceutical composition may be administered by conventional routes, including (but not limited to): oral, respiratory, intratumoral, intraperitoneal, intravenous, or topical administration.

The pharmaceutical composition of the present invention can be directly used to bind to the respiratory syncytial virus fusion protein (preferably the pre-fusion F protein) molecules, and thus can be used to prolong the half-life of the drug. In addition, other therapeutic agents can also be used at the same time.

The pharmaceutical composition according to the present invention comprises a safe and effective amount (e.g., 0.001-99 wt %, preferably 0.01-90 wt %, preferably 0.1-80 wt %) of the monoclonal antibody according to the present invention (or a conjugate thereof) and a pharmaceutically acceptable carrier or excipient. Such carriers include (but are not limited to): saline, buffers, glucose, water, glycerol, ethanol, and a combination thereof. A pharmaceutical preparation should correspond to the administration mode. The pharmaceutical composition according to the present invention can be prepared in the form of an injection, for example, by a conventional method using physiological saline or an aqueous solution containing glucose and other adjuvants. A pharmaceutical composition, for example, an injection and a solution, should be prepared under aseptic conditions. The administration amount of an active ingredient is a therapeutically effective amount, for example, about 1 $\mu$g per kilogram of body weight to about 5 mg per kilogram of body weight daily. In addition, the polypeptide according to the present invention may also be used in combination with an additional therapeutic agent.

When a pharmaceutical composition is used, a safe and effective amount of immunoconjugate is administered to a mammal, wherein the safe and effective amount is generally at least about 10 $\mu$g per kilogram of body weight, and in most cases, no more than about 8 mg per kilogram of body weight, preferably, the amount is from about 10 $\mu$g per kilogram of body weight to about 1 mg per kilogram of body weight. Of course, a specific amount should also depend on the factors such as administration route and physical conditions of a patient, which fall into the skills of skilled physicians.

**Use for detection and the kit**

The antibody of the present invention can be used for detection, for example, for detection of samples to provide diagnostic information.

In the present invention, the specimens (samples) used include cells, tissue samples and biopsy specimens. The term "biopsy" used in the present invention shall include all kinds of biopsy known to those skilled in the art. Therefore, the biopsy used in the present invention may include, for example, tissue samples prepared by endoscopic methods or organ puncture or needle biopsy.

The samples used in the present invention include fixed or preserved cells or tissue samples.

The present invention also provides a kit containing the antibody (or a fragment thereof) of the present invention. In a preferred embodiment of the present invention, the kit further includes a container, instructions for use, buffer, and the like. In a preferred embodiment, the antibody of the present invention can be immobilized on a detection plate.

**The main advantages of the present invention include:**

The present invention provides the design and use of a fully human antibody for neutralizing the respiratory syncytial virus, which has the following advantages:

1) The amino acid sequence of the antibody of the present invention has relatively less somatic mutation sites and low immunogenicity.
2) Affinity constants of the antibody of the present invention for F proteins of different subtypes of RSV reach picomolar level and are superior to that of the two clinically available antibodies.
3) The antibody of the present invention is effective in preventing RSV infection and inhibiting the spread of RSV after infection in *in vitro* and *in vivo* experiments.
4) The study of the optimized novel antibody of the present invention provides a clearer understanding of the epitopes it recognizes and the mechanism of virus neutralization, which provides new targets for drug design.

The present invention is further illustrated below in conjunction with specific embodiments. It should be understood that the examples are not intended to limit the scope of the invention. The experimental methods in the following examples which do not specify the specific conditions are usually in accordance with conventional conditions, such as conditions described in Sambrook et al., Molecular Cloning: Laboratory Manual (New York: Cold Spring Harbor Laboratory Press, 1989), or in accordance with the conditions recommended by the manufacturer. Unless otherwise stated, percentages and parts are by weight.

**Example 1 Modeling of 4F1 and RSV pre-F interactions using homology modeling and molecular docking techniques**

**1.1 Modeling of 4F1 and RSV pre-F interactions using homology modeling and molecular docking technique**

Computer-aided techniques were used to optimize and modify a broad-spectrum neutralizing antibody 4F1 isolated from human PBMC in the previous stage. Firstly, homology modeling of the 4F1 antibody was performed using the antibody modeling module in Prime docking (Schro"dinger, Inc.). Briefly, the heavy and light chain sequences were inputted to the program. The framework template (FRH/FRL region) was identified by searching in antibody structure database, and the antibody structure with the highest similarity to the sequence was determined as the template. In this example, the 4F1 variable region sequence was aligned to the human antibody germline gene database, and the IGHV3/IGKV2 sequence with high homology was selected as the design template. The antibody CDR regions were grafted onto the template, and then the antibody structure model was constructed. The homology modeling structure of 4F1 was docked onto the structure of pre-F (4JHW) using RosettaDock. The structure of the complex was determined based on the results of the point mutation of antibody and antigen and the docking score.

**Result:** The structure figure was generated using the PyMOL molecular graph system (version 2.0, Schrödinger, LLC) (Fig. 1A).

## 1.2 Optimization design of antibodies

Based on the key amino acids associated with the antibody structure and the key positions of interaction with pre-F demonstrated in the docking structural model of the complex of 4F1 and pre-F, reverse mutation sites were designed in the Framework regions, and mutation sites that may enhance antibody affinity were introduced at the sites where 4F1 binds to the antigen.

Immunogenicity has been a major concern for therapeutic antibodies. Studies have reported that a higher somatic mutation frequency would increase the risk of antibody immunogenicity. Therefore, reversing somatic mutation sites back to the sequence of the inferred germline (iGL) may reduce the immunogenicity of the antibody. Based on the structural analysis, in order to reduce the immunogenicity that might be present in the 4F1 antibody, 9 somatic mutation sites in the heavy chain frame region sequence and 7 somatic mutation sites in the light chain were simultaneously reversed to the corresponding germline sequence (Fig. 1B), thereby a 4F1 germline reversion was obtained.

High affinity is usually an important indicator for quality assessment of antiviral antibodies. In order to further improve the affinity of 4F1 antibody, the present project optimizes the affinity of the antibody by using Schrödinger. Five mutation sites (VH-S75R, VH-V102S, VL-L30R, VL-S32Q and VL-S72Q) were introduced into the heavy chain and light chain of 4F1 germline reversion, respectively, which were named 4F1En1, 4F1En2, 4F1En3, 4F1En4 and 4F1En5.

**Result:** The sequence alignment results are shown in Fig. 2. The amino acid and nucleotide sequences are shown in Table 1.

Table 1 Sequences of antibody variable regions

| Name | Sequence | SEQ ID NO: |
|---|---|---|
| Amino acid sequence of the heavy chain variable region of 4F1 germline reversion | QVQLVESGGGVVQPGRSLRLSCAASGFSFYSYSVHWVRQAPGKGLEWVADVVYDGNHQHYTDSVKGRFTISRDTSKNTVYLQMGSLRAEDTALYYCTARSLVITLAGAGRDDYWGQGTTVTVSSAS | 1 |
| Amino acid sequence of the heavy chain variable region 4F1En1 | QVQLVESGGGVVQPGRSLRLSCAASGFSFYSYSVHWVRQAPGKGLEWVADVVYDGNHQHYTDSVKGRFTISRDTRKNTVYLQMGSLRAEDTALYYCTARSLVITLAGAGRDDYWGQGTTVTVSSAS | 2 |
| Amino acid sequence of the heavy chain variable region 4F1En2 | QVQLVESGGGVVQPGRSLRLSCAASGFSFYSYSVHWVRQAPGKGLEWVADVVYDGNHQHYTDSVKGRFTISRDTRKNTVYLQMGSLRAEDTALYYCTARSLSITLAGAGRDDYWGQGTTVTVSSAS | 3 |
| Amino acid sequence of the light chain variable region of 4F1 germline reversion | DIVMTQSPLSLPVTPGEPASISCRSSQSLLHSNGYTYLDWYLQKPGQSPQLLIFLGSSRASGVPDRFSGSGSGTDFTLKISRVEAEDVGVYYCVQDLQTSLTFGGGTKLEIKRT | 4 |

(continued)

| Name | Sequence | SEQ ID NO: |
|------|----------|------------|
| Amino acid sequence of the light chain variable region 4F1En3 | DIVMTQSPLSLPVTPGEPASISCRSSQSLRHSNGYT YLDWYLQKPGQSPQLLIFLGSSRASGVPDRFSGSG SGTDFTLKISRVEAEDVGVYYCVQDLQTSLTFGG GTKLEIKRT | 5 |
| Amino acid sequence of the light chain variable region 4F1En4 | DIVMTQSPLSLPVTPGEPASISCRSSQSLLHQNGYT YLDWYLQKPGQSPQLLIFLGSSRASGVPDRFSGSG SGTDFTLKISRVEAEDVGVYYCVQDLQTSLTFGG GTKLEIKRT | 6 |
| Amino acid sequence of the light chain variable region 4F1En5 | DIVMTQSPLSLPVTPGEPASISCRSSQSLLHSNGYT YLDWYLQKPGQSPQLLIFLGSSRASGVPDRFSGSG QGTDFTLKISRVEAEDVGVYYCVQDLQTSLTFGG GTKLEIKRT | 7 |
| Nucleotide sequence of the heavy chain variable region of 4F1 germline reversion | CAGGTGCAGCTCGTGGAGTCTGGCGGCGGCGTG GTGCAGCCAGGCCGGAGCCTCAGACTGTCTTGC GCTGCCAGCGGCTTCAGCTTCTACTCTTACTCCG TGCACTGGGTGAGACAGGCTCCAGGCAAGGGCT TGGAGTGGGTGGCTGACGTGGTGTACGACGGCA ACCACCAGCACTACACCGACTCTGTGAAGGGCA GATTCACAATTAGCAGGGACACATCCAAGAACA CAGTGTACCTACAAATGGGCTCCCTGAGAGCCG AGGACACCGCCCTGTACTACTGCACCGCCCGAT CGCTCGTGATCACCCTCGCTGGCGCTGGCCGGG ACGACTACTGGGGCCAGGGCACAACAGTGACC GTGTCTTCCGCGTCGAC | 8 |

(continued)

| Name | Sequence | SEQ ID NO: |
|------|----------|------------|
| Nucleotide sequence of the heavy chain variable region 4F1En1 | CAGGTGCAGCTCGTGGAGTCTGGCGGCGGCGTG GTGCAGCCAGGCCGGAGCCTCAGACTGTCTTGC GCTGCCAGCGGCTTCAGCTTCTACTCTTACTCCG TGCACTGGGTGAGACAGGCTCCAGGCAAGGGCT TGGAGTGGGTGGCTGACGTGGTGTACGACGGCA ACCACCAGCACTACACCGACTCTGTGAAGGGCA GATTCACAATTAGCAGGGACACAAGAAAGAAC ACAGTGTACCTACAAATGGGCTCCCTGAGAGCC GAGGACACCGCCCTGTACTACTGCACCGCCCGA TCGCTCGTGATCACCCTCGCTGGCGCTGGCCGG GACGACTACTGGGGCCAGGGCACAACAGTGAC CGTGTCTTCCGCGTCGAC | 9 |
| Nucleotide sequence of the heavy chain variable region 4F1En2 | CAGGTGCAGCTCGTGGAGTCTGGCGGCGGCGTG GTGCAGCCAGGCCGGAGCCTCAGACTGTCTTGC GCTGCCAGCGGCTTCAGCTTCTACTCTTACTCCG TGCACTGGGTGAGACAGGCTCCAGGCAAGGGCT TGGAGTGGGTGGCTGACGTGGTGTACGACGGCA ACCACCAGCACTACACCGACTCTGTGAAGGGCA GATTCACAATTAGCAGGGACACAAGAAAGAAC ACAGTGTACCTACAAATGGGCTCCCTGAGAGCC GAGGACACCGCCCTGTACTACTGCACCGCCCGA TCGCTCAGCATCACCCTCGCTGGCGCTGGCCGG GACGACTACTGGGGCCAGGGCACAACAGTGAC CGTGTCTTCCGCGTCGAC | 10 |
| Nucleotide sequence of the light chain variable region of 4F1 germline reversion | GACATCGTGATGACCCAGTCCCCACTGAGCCTC CCTGTGACACCAGGCGAGCCCGCTTCTATCTCTT GCCGGTCTTCTCAGAGCCTGCTGCACTCTAACG GCTACACATACCTCGACTGGTATCTCCAGAAGC CAGGCCAGTCTCCACAGCTGCTCATCTTCCTCGG CAGCTCTAGGGCCAGCGGCGTGCCCGACCGCTT CTCCGGCTCTGGCTCTGGCACCGACTTCACACTC AAGATCAGCAGAGTGGAGGCCGAGGACGTGGG CGTGTACTACTGCGTGCAGGACCTCCAGACATC CCTGACATTCGGCGGCGGCACAAAGCTGGAGAT CAAGcgtacg | 11 |

(continued)

| Name | Sequence | SEQ ID NO: |
|------|----------|------------|
| Nucleotide sequence of the light chain variable region 4F1En3 | GACATCGTGATGACCCAGTCCCCACTGAGCCTC CCTGTGACACCAGGCGAGCCCGCTTCTATCTCTT GCCGGTCTTCTCAGAGCCTGAGGCACTCTAACG GCTACACACCTCGACTGGTATCTCCAGAAGC CAGGCCAGTCTCCACAGCTGCTCATCTTCCTCGG CAGCTCTAGGGCCAGCGGCGTGCCCGACCGCTT CTCCGGCTCTGGCTCTGGCACCGACTTCACACTC AAGATCAGCAGAGTGGAGGCCGAGGACGTGGG CGTGTACTACTGCGTGCAGGACCTCCAGACATC CCTGACATTCGGCGGCGGCACAAAGCTGGAGAT CAAGcgtacg | 12 |
| Nucleotide sequence of the light chain variable region 4F1En4 | GACATCGTGATGACCCAGTCCCCACTGAGCCTC CCTGTGACACCAGGCGAGCCCGCTTCTATCTCTT GCCGGTCTTCTCAGAGCCTGCTGCACCAGAACG GCTACACACCTCGACTGGTATCTCCAGAAGC CAGGCCAGTCTCCACAGCTGCTCATCTTCCTCGG CAGCTCTAGGGCCAGCGGCGTGCCCGACCGCTT CTCCGGCTCTGGCTCTGGCACCGACTTCACACTC AAGATCAGCAGAGTGGAGGCCGAGGACGTGGG CGTGTACTACTGCGTGCAGGACCTCCAGACATC CCTGACATTCGGCGGCGGCACAAAGCTGGAGAT CAAGcgtacg | 13 |
| Nucleotide sequence of the light chain variable region 4F1En5 | GACATCGTGATGACCCAGTCCCCACTGAGCCTC CCTGTGACACCAGGCGAGCCCGCTTCTATCTCTT GCCGGTCTTCTCAGAGCCTGCTGCACTCTAACG GCTACACACCTCGACTGGTATCTCCAGAAGC CAGGCCAGTCTCCACAGCTGCTCATCTTCCTCGG CAGCTCTAGGGCCAGCGGCGTGCCCGACCGCTT CTCCGGCTCTGGCCAGGGCACCGACTTCACACT CAAGATCAGCAGAGTGGAGGCCGAGGACGTGG GCGTGTACTACTGCGTGCAGGACCTCCAGACAT CCCTGACATTCGGCGGCGGCACAAAGCTGGAGA TCAAGcgtacg | 14 |
| Amino acid sequence of the heavy chain variable region of 4F1 WT | EVQLVQSGGGVVRPGRSLRLSCAASGFSFYSYSV HWVRQAPGKGLEWVADVVYDGNHQHYTESVRG RFSISRDTSTNTVYLQMGSLRPEDTALYYCTARSL VITLAGAGRDDYWGQGTRVTVSS | 15 |

(continued)

| Name | Sequence | SEQ ID NO: |
|---|---|---|
| Amino acid sequence of the light chain variable region of 4F1 WT | DIVMTQSPLSLSVTPGEPASISCKSSQSLLHSNGYT YLDWYLQKPGKSPQLLIFLGSSRASGVPARFSGSG SGTDFTLEISRVEAEDVGVYYCVQDLQTSLTFGGG TKVDIK | 16 |

Note: the heavy and light chain CDR1, CDR2, CDR3 sequences are underlined in order.

**Example 2 Expression and purification of antibodies**

CHO cells were transiently transfected to express the fully human antibodies. One day prior to transfection (day -1), ExpiCHO-S™ cells were subseeded at a final density of $3\times10^6$-$4\times10^6$ viable cells/mL and cells were allowed to grow overnight. The following day (day 0), viable cell density and survival rate were determined. Before transfection, the cell density should be approximately $7\times10^6$-$10\times10^6$ viable cells/mL, and the survival rate should be 95-99%. The cells were diluted to a final density of $6\times10^6$ viable cells/mL. ExpiFectamine™ CHO/plasmid DNA complex was prepared using pre-cooled reagents (4 °C). The ExpiFectamine™ CHO/plasmid DNA complex was incubated at room temperature for 1-5 minutes, then the solution was slowly transferred to a CHO cell culture flask, while the flask was gently shaken during addition. The cells were incubated on an orbital shaker (in an incubator at 37 °C containing 8% CO2 under humidified air). After 7-11 days of incubation, the supernatant was collected to purify the antibody when half of the cells were dead.

Antibodies were purified using Protein G Agarose 4FF filler (purchased from GE). Firstly, the collected CHO cell suspension was centrifuged at 4000 rpm at 4 °C for 30 min, and then the collected supernatant was filtered by a 0.45 um filter for purification. The Protein G Agarose 4FF filler was added in a gravity centrifugation column, and stabilized with 3 times the column volume of 20% ethanol. The column was then equilibrated with 5 times the column volume of binding buffer. The sample was loaded, and the column was then equilibrated with 10 times the column volume of binding buffer, and finally 3 times the column volume of elution buffer was used to elute the column. Neutralization buffer was added to the eluted antibody solution to allow the pH to reach about 7.5. The antibody solution was dialyzed in 5L 1×PBS for 3 times, then the antibodies could be concentrated and stored at -80 °C.

**Example 3 ELISA detection of antigen binding activity of antibodies**

Whether the expressed antibodies recognize RSV A2 and B9320 pre-fusion F proteins was detected by ELISA. The A2 F protein sequence refers to UniProtKB/Swiss-Prot: P03420.1, the B9320 sequence refers to UniProtKB/Swiss-Prot: Q6V2E7, the RSV pre-fusion F protein was designed according to the strategy adopted by Jason S. McLellan. Science 2013, and the RSV post-fusion F protein was designed according to the strategy adopted by Davide Corti. Nature 2013. The whole gene synthesis was performed by Shanghai Generay Biotech Co., Ltd. The genes were constructed into invitrogen pcDNA3.1 expression vectors and expressed by the mammalian cell CHO expression system, referring to Invitrogen ExpiCHO-S™ Expression System manual. Palivizumab (SYNAGIS®) was used as the positive control antibody, which was purchased from Abbott. ELISA plate was coated with 5 μg/mL of F protein at 100 μL per well and incubated at 4 °C overnight. The plate was washed 3 times with PBST on the following day. The plate was blocked with 100 μL 2% BSA per well at 37 °C for 2 h, then washed with PBST for 3 times. Samples were loaded at 100 μL per well and incubated at 37 °C for 2 h. The plate was washed again with PBST for 3 times. Goat Anti-Human IgG (Fc specific)-Peroxidase antibody was diluted at 1:2000, added to the plate at 100 μL per well, and incubated at 37 °C for 1 h. The plate was washed with PBST for 3 times. The substrate TMB was added to the plate at 100 μL/well for color development, and the reaction could be performed in dark at 37 °C for 15min if the color is light. 2M $H_2SO_4$ was added at 50 μL per well to terminate the reaction. The determination of OD450 value and data processing were performed.

**Result:** Compared with 4F1, 4F1 with reverse mutations (germline reversion) maintains the same binding and neutralizing activities as 4F1 wild type. 4F1En1 (VH-S75R) has higher binding activity compared to 4F1WT (Fig. 1C).

**Example 4 Affinity constants of antibodies detected by biofilm layer interference technique**

To investigate the affinity and neutralizing activity of the 4F1En1 antibody in comparison to existing clinically approved or clinically evaluated antibodies, the affinity of the 4F1En1 antibody for two different isoforms of RSV pre-F protein was analyzed by using ForteBio Octet® RED96 Interaction Analyzer. Palivizumab and MEDI8897 served as controls. This assay detected the binding constants (represented as KD values) of different forms of antibodies for pre-F proteins derived from RSV/A/A2 and RSV/B/B9320 virus strains. ForteBio Octet® RED96 Interaction Analyzer was employed in this experiment for analysis. Kinetics buffer (KB:1× PBS, 0.01% BSA) was used as a buffer in this experiment. The AHC sensor was first immersed into a black 96-well plate containing KB for at least 10 min; 5 μg/ml monoclonal antibody prepared in KB was captured to the surface of biosensor coated with anti-human IgG Fc (AHC) for 5 min. The baseline was measured in KB, and then the sensor was immersed in a well containing recombinant DS-Cav1 diluted in KB for 600 s (association phase). The sensor was then immersed in KB for an indicated time (dissociation phase) up to 1200 s. Finally, the results were analyzed by software. Average Kon, Koff and KD values were calculated using a 1:1 binding global fitting kinetic model with an $R^2$ value of ≥0.95.

Result: The optimized 4F1En1 antibody showed higher affinity to pre-F of RSV type A and B than the existing antibody. Compared to 4F1WT, 4F1En1 had a higher association constant for A2-preF and a slower dissociation constant for B9320-pre-F. In addition, 4F1En1 had at least 10-fold higher affinity for A2-preF and at least 100-fold higher binding affinity for B9320/preF compared to PVZ and MEDI8897. The neutralization potency of 4F1En for RSV/A/A2 strain was increased by 3 times compared to 4F1-WT. In addition, the IC50 value of 4F1 against RSV/A2 is 62-fold lower than that of pavlizumab, and similar to that of MEDI8897 (Figs. 3A-D).

**Example 5 Virus micro-neutralization assay**

**5.1 Virus TCID$_{50}$ assay**

The titer of the diluted virus solution in the RSV micro-neutralization assay was verified by TCID$_{50}$ assay. 200 μl of diluted virus solution was added to wells B2-D2 of a 96-well cell plate and 100 μl of cell culture solution was added to other wells. 100 μl of virus solution was aspirated from wells B2-D2 and added to wells B3-D3, mixed well and subjected to a 2-fold serial dilution. The virus solution was diluted for a total of 18 concentration points, with 3 replicate wells. Subsequently, the test plates were incubated at 37°C in a 5% CO2 incubator for 2 hours. HEp2 cells were inoculated into the test plates at a density of 25,000 cells per well and incubated at 37°C in a 5% CO2 incubator for 5 days.

After 5 days of incubation, the supernatant was discarded and the cells in the test plates were fixed with 80% acetone. The viral content in the cells was detected by ELISA. The raw data were used for virus TCID$_{50}$ calculation (Karber method). The calculation formula is as follows:

$$TCID_{50}/well = Antilog10[(number\ of\ positive\ wells/3)-0.5\}\times0.3]/2$$

Positive well determination criteria: reading value of test well > (average value of culture control + 3 × SD value of culture medium control)

QC standard: the titer of diluted virus solution shall be 50-2000 TCID$_{50}$/well.

**5.2 Virus micro-neutralization assay**

The virus strains to be tested, A2 and B9320 (purchased from ATCC), were diluted to 4000 TCID$_{50}$/ml with cell culture medium, respectively. Fold-diluted antibodies and 200 TCID$_{50}$/well of virus were added into 96-well cell culture plates in equal volume ratios, mixed well, and then incubated for 2 hours at 37 °C in a 5% CO2 incubator. Subsequently, HEp2 cells were inoculated into the test plates at a density of 25,000 cells per well and incubated for 5 days at 37 °C in a 5% CO2 incubator. Antibodies were tested at 9 concentrations in 3-fold gradient dilution, with 3 replicate wells, with a starting test concentration of 4000 ng/ml.

After 5 days of incubation, the supernatant was discarded and the cells in the test plates were fixed with 80% acetone. The viral content of the cells was detected by ELISA. The raw data were used for the calculation of the neutralizing activity of the antibody at different concentrations. The calculation formula is as follows:

Percentage of activity (%) = (reading value of test well - mean value of virus control)/(mean value of cell control - mean value of virus control) × 100

EC$_{50}$ values were calculated by Prism software and the neutralization activity curve was fitted by sigmoidal dose-response (variable slope) method.

**Results:** The results of the *in vitro* micro-neutralization assay confirmed that 4F1En1 (VH-S75R) had higher neutralization activity compared to 4F1WT (Figs. 1D-E).

**Example 6 Inhibitory function of neutralizing antibodies against the infection and spread of RSV detected by content cell imaging**

HEp-2 cells were spread at 10000 per well in a 96-well black culture plate and incubated overnight at 37 °C in an incubator for 1 day. To verify the neutralization ability of the optimized 4F1 antibodies against the virus, a series of dilutions of the antibodies were incubated with 500 pfu RSV/A2 for 1 hour and added to Hep-2 cells. To verify whether the antibodies are able to inhibit the spread of RSV after infecting cells, firstly, the RSV viruses were inoculated into HEp-2, and after 24 hours of infection, the supernatant was discarded, and series diluted antibodies were added to the infected HEp-2. After 3 days of virus infection, the cells were fixed for 15 min in paraformaldehyde and blocked for 30 min with 3% bovine serum albumin (BSA) in PBS. Biotinylated anti-RSV-F and anti-RSV-G monoclonal antibodies were diluted to 1 μg/mL in 3% BSA in PBS and used for the detection of RSV infection through incubation for 2 h at room temperature. FITC-coupled streptavidin was diluted at 1:500 in 0.5% BSA in PBS, and DAPI (1:1000) was co-incubated with the secondary antibody to stain the nuclei. The staining was performed for 1 h at room temperature. To remove the unbound antibodies, cells were washed 5 minutes, 3 times in PBS containing 0.05% Tween-20. The 96-well plate was photographed and data was counted using the PerkinElmer Opera Phenix™ High Content Screening System. Cells were photographed in 2 fluorescence channels using the 2× or 10× objective lens. 10 different regions were imaged using the 10× objective lens. To determine how many cells were infected with RSV, the number of RSV-positive cells was divided by the total number of nuclei and multiplied by 100 to obtain the percentage of RSV-infected cells.

**Results:** This project further tested the ability of 4F1En1 to block virus spread between cells. In this assay, serial dilutions of 4F1-En, Palivizumab, and MEDI887 were added 1 h prior to infection (to inhibit infection) or 24 h after infection (to inhibit spread). IC$_{50}$ values were calculated after 72 h of infection using high content analysis. As shown in Fig. 4, 4F1En1 not only inhibited viral infection, but also inhibited viral spread at 24 h after HEp-2 cells were pre-infected with RSV/A2. 4F1En1 also limited the size of syncytium formation (Fig. 4C). The inhibition function of viral infectivity and spread of 4F1 was comparable to that of MEDI8897 and significantly more potent than Palivizumab (Figs. 4D-E). To sum up, these results suggest that 4F1 En inhibits viral infection as well as viral spread and syncytium formation.

**Example 7 Challenging experiment**

In this example, the ability of antibody 4F1 of the present invention and Palivizumab to prevent RSV infection in mice was tested. The method is as follows:

6-8-week-old BalB/c female mice were placed in advance into a biosafety level II laboratory animal experiment room. On day 0, mice were injected intraperitoneally with 15 mg/kg and 1.5 mg/kg of 4F1 antibody, Palivizumab and PBS, respectively. After 24h, mice were anesthetized with sodium pentobarbital and subjected to intranasal challenge with 5*10$^5$ PFU/50μl/mouse of RSV A2 virus. Animals were killed 4 days later, and their lungs and nasal turbinates were collected. The left lung was removed from each mouse and fixed with 4% paraformaldehyde, embedded in paraffin and stained routinely with hematoxylin-eosin (HE). The pathological damage and inflammatory cell infiltration of lungs were visualized by using a digital section scanning system. The right lung tissue and nasal turbinate of each mouse were taken for tissue homogenization. Total RNA was extracted with the EZ-press RNA Purification Kit and then reverse transcribed to cDNA. The genome of RSV was relatively quantified by real-time quantitative PCR (RT-PCR). The primers were designed based on conserved fragments selected through alignments of the gene sequences coding the NP protein of RSV. Mouse β-actin was used as an internal reference gene (see Table 2). Toyobo KOD SYBR®qPCR Mix instruction was referred.

Table 2 Primers for real-time quantitative RT-PCR

| Amplification primer | 5'-3' Sequence | SEQ ID NO: |
|---|---|---|
| RSV NP -sp | AGATCAACTTCTGTCATCCAGCAA | 17 |
| RSV NP -asp | TTCTGCACATCATAATTAGGAGTATCAAT | 18 |
| mouse β-actin-sp (internal reference) | GGCTGTATTCCCCTCCATCG | 19 |
| mouse β-actin-asp (internal reference) | CCAGTTGGTAACAATGCCATGT | 20 |

**Results:** Result of real-time quantitative RT-PCR is shown in Fig. 5B, indicating that 4F1 was significantly more effective than palivizumab monoclonal antibody in reducing HRSV virus in lung and nasal turbinate, and was effective at the concentration as low as 1.5 mg per kg of body weight. Lung histopathology results showed that 4F1 prevented monocyte infiltration into the lung interstitium and alveolar spaces, which was superior to Palivizumab (Fig. 5C). These data suggest that 4F1En1 exhibits highly effective protection *in vivo* and would be a promising candidate for the prevention of RSV infection.

All documents mentioned in the present invention are incorporated by reference herein as if each document were incorporated separately by reference. Furthermore, it should be understood that after reading the foregoing teachings of the invention, various changes or modifications may be made to the invention by those skilled in the art and that these equivalents are equally within the scope of the claims appended to this application.

**Claims**

1. An antibody specific to respiratory syncytial virus fusion protein, wherein the antibody comprises a heavy chain and a light chain,

   wherein, the variable region of the heavy chain comprises the following amino acid mutations corresponding to a sequence as shown in SEQ ID NO: 15:

   (Z1) n reverse mutations selected from the following group, wherein n is 3, 4, 5, 6, 7, 8 or 9:

   position 1, E→Q
   position 6, Q→E
   position 13, R→Q
   position 62, E→D
   position 65, R→K
   position 69, S→T
   position 76, T→K
   position 88, P→A
   position 119, R→T;

   (Z2) a neutralization-enhanced FR mutation
   position 75, S→R;
   (Z3) a combination of Z1 and Z2; and

   the variable region of the light chain comprises the following amino acid mutations corresponding to a sequence as shown in SEQ ID NO: 16:

   (Y1) m reverse mutations selected from the following group, wherein m is 3, 4, 5, 6 or 7:

   position 12, S→P
   position 24, K→R
   position 47, K→Q
   position 65, A→D
   position 79, E→K
   position 109, V→L
   position 110, D→E;

   (Y2) a neutralization-enhanced CDR mutation
   position 30, L→R;
   (Y3) a combination of Y1 and Y2.

2. The antibody according to claim 1, wherein the antibody specifically binds to the respiratory syncytial virus pre-fusion F protein.

3. The antibody according to claim 1, wherein the variable region of the heavy chain is selected from the group consisting of:

a) a heavy chain variable region of which the amino acid sequence is shown in SEQ ID NO: 1 or 2; and

b) a heavy chain variable region as shown in a derived sequence of the amino acid sequence as shown in SEQ ID NO: 1 or 2, which is obtained by maintaining 3 CDR regions and substituting, deleting, modifying and/or adding at least one (e.g., 1-5, preferably 1-3, and preferably 1 or 2) amino acid residue in FR regions, and retains a binding affinity to the respiratory syncytial virus fusion protein (preferably the pre-fusion F protein).

4. The antibody according to claim 1, wherein the variable region of the light chain is selected from the group consisting of:

c) a light chain variable region of which the amino acid sequence is shown in SEQ ID NO: 4 or 5; and

d) a heavy chain variable region as shown in a derived sequence of the amino acid sequence as shown in SEQ ID NO: 4 or 5, which is obtained by maintaining 3 CDR regions and substituting, deleting, modifying and/or adding at least one (e.g., 1-4, preferably 1-3, and preferably 1 or 2) amino acid residue in FR regions, and retains a binding affinity to the respiratory syncytial virus fusion protein (preferably the pre-fusion F protein).

5. The antibody according to claim 1, wherein the variable region of the heavy chain has an amino acid sequence as shown in SEQ ID NO: 1 or 2.

6. The antibody according to claim 1, wherein the variable region of the light chain has an amino acid sequence as shown in SEQ ID NO: 4 or 5.

7. The antibody according to claim 1, wherein the antibody comprises a heavy chain variable region of which the sequence is shown in SEQ ID NO: 2 and a light chain variable region of which the sequence is shown in SEQ ID NO: 4.

8. A recombinant protein comprising:

(i) the antibody according to claim 1; and
(ii) an optional tag sequence to assist expression and/or purification.

9. A CAR construct, wherein the scFv fragment of the antigen-binding domain of the CAR construct is a binding region specifically binding to the RSV fusion protein (preferably the pre-fusion F protein), and the scFv comprises the variable region of the heavy chain and the variable region of the light chain as described in claim 1.

10. A recombinant immune cell which expresses an exogenous CAR construct according to claim 7.

11. An antibody-drug conjugate comprising:

(a) an antibody moiety, which is selected from the group consisting of the antibody according to claim 1, and a combination thereof; and
(b) a coupling moiety coupled to the antibody moiety, which is selected from the group consisting of a detectable label, a drug, a toxin, a cytokine, a radionuclide, an enzyme, and a combination thereof.

12. A pharmaceutical composition which comprises:

(i) an active ingredient, wherein the active ingredient is selected from the group consisting of: the antibody according to claim 1, the recombinant protein according to claim 6, the immune cell according to claim 8, the antibody-drug conjugate according to claim 9, and a combination thereof; and
(ii) a pharmaceutically acceptable carrier.

13. A polynucleotide encoding a polypeptide selected from the group consisting of:

(1) the antibody according to any one of claims 1-7; and
(2) the recombinant protein according to claim 8;
(3) the CAR construct according to claim 9.

14. A method for detection of respiratory syncytial virus in a sample, which comprises steps of:

(1) contacting the sample with the antibody according to any one of claims 1-7;

(2) detecting whether an antigen-antibody complex is formed, wherein the formation of the complex indicates the presence of respiratory syncytial virus in the sample.

15. A method for treating infection of respiratory syncytial virus, which comprises: administering the antibody according to claims 1-7, an antibody-drug conjugate of the antibody, or a CAR-T cell expressing the antibody, or a combination thereof to a subject in need thereof.

A

B

C

RSV/A/A2 pre-F

RSV/B/B9320 pre-F

D

RSV/A/A2

- 4F1 WT
- 4F1 germline reversion
- 4F1En1
- 4F1En2
- 4F1En3
- 4F1En4
- 4F1En5

E

| mAbs | RSV A2 pre F | | RSV B9320 pre F | | RSV A2 Neutralizing activity | |
|---|---|---|---|---|---|---|
| | $EC_{50}$ ng/ml | Fold change | $EC_{50}$ ng/ml | Fold change | $IC_{50}$ ng/ml | Fold change |
| 4F1-WT | 52.59 | 1.00 | 111.30 | 1.00 | 12.17 | 1.00 |
| 4F1 germline reversion | 53.65 | 1.02 | 78.13 | 0.70 | 5.37 | 0.44 |
| 4F1En1 | 24.52 | 0.47 | 56.45 | 0.51 | 4.05 | 0.33 |
| 4F1En2 | 56.08 | 1.07 | 227.50 | 2.04 | 22.17 | 1.82 |
| 4F1En3 | 35.89 | 0.68 | 58.54 | 0.53 | 8.49 | 0.70 |
| 4F1En4 | 37.49 | 0.71 | 60.58 | 0.54 | 18.04 | 1.48 |
| 4F1En5 | 54.81 | 1.04 | 77.29 | 0.69 | 22.74 | 1.87 |

Fig. 1

```
                      10            20            30            40            50            60
4F1VH GL              QVQLVESGGGVVQPGRSLRLSCAASGFTFSSYAMHWVRQAPGKGLEWVAVISYDGSNKYYAD
4F1 WT                E····Q······R···········S·Y··SV·················DVV···NHQH·TE
4F1 germline reversion ····················S·Y··SV·················DVV···NHQH·T·
4F1En1                ····················S·Y··SV·················DVV···NHQH·T·
4F1En2                ····················S·Y··SV·················DVV···NHQH·T·

                      70            80            90            100           110           120
4F1VH GL              SVKGRFTISRDNSKNTLYLQMNSLRAEDTAVYYCAARSLVITFGGAGRDDYWGQGTTVTVSS
4F1 WT                ··R···S····T·T··V····G···P···L···T·······LA·······R····
4F1 germline reversion ··········T···V····G·········L···T·······LA···········
4F1VHEn1              ·········TR··V····G·········L···T·······LA···········
4F1VHEn2              ·········T···V····G·········L···T···S·LA···········

                      10            20            30            40            50            60
4F1VK GL              DIVMTQSPLSLPVTPGEPASISCRSSQSLLHSNGYNYLDWYLQKPGQSPQLLIYLGSNRASG
4F1 WT                ···········S··········K····T············K·····F··S····
4F1 germline reversion ·····················T············F··S····
4F1En3                ······················R····T··············F··S····
4F1En4                ······················Q···T··············F··S····
4F1En5                ···························T··············F··S····

                      70            80            90            100           110
4F1VK GL              VPDRFSGSGSGTDFTLKISRVEAEDVGVYYCMQALQTSLTFGGGTKLEIK
4F1 WT                ··A············E··············V·D·····VD··
4F1 germline reversion ·····························V·D·······
4F1En3                ·····························V·D·····
4F1En4                ·····························V·D·····
4F1En5                ·········Q···················V·D·····
```

# Fig. 2

Fig. 3

Fig. 4

Fig. 5

## INTERNATIONAL SEARCH REPORT

| International application No. |
| --- |
| **PCT/CN2022/123876** |

| A. | CLASSIFICATION OF SUBJECT MATTER |
| --- | --- |

C07K 16/10(2006.01)i; C12N 15/11(2006.01)i; C12N 5/07(2010.01)i; C07K 19/00(2006.01)i; A61K 39/42(2006.01)i; A61P 31/14(2006.01)i; A61P 11/06(2006.01)i

According to International Patent Classification (IPC) or to both national classification and IPC

| B. | FIELDS SEARCHED |
| --- | --- |

Minimum documentation searched (classification system followed by classification symbols)

C07K; C12N; A61K; A61P

Documentation searched other than minimum documentation to the extent that such documents are included in the fields searched

Electronic data base consulted during the international search (name of data base and, where practicable, search terms used)

CNABS; CNTXT; DWPI; WPABS; ENTXT; OETXT; CNKI; WEB OF SCIENCE: 呼吸道合胞病毒, 抗体, 融合蛋白, 嵌合抗原受体, F蛋白, 突变, rspiratory syncytial virus, RSV, antibody, fusion protein, car-t, car, F protein, mutation; 中国专利生物序列检索系统, China Patents Biological Sequence Search System; Genbank和检索的序列: SEQ ID NOs: 1-20.

| C. | DOCUMENTS CONSIDERED TO BE RELEVANT |
| --- | --- |

| Category* | Citation of document, with indication, where appropriate, of the relevant passages | Relevant to claim No. |
| --- | --- | --- |
| A | CN 111606993 A (SHANGHAI INSTITUTES FOR BIOLOGICAL SCIENCES, CHINESE ACADEMY OF SCIENCES) 01 September 2020 (2020-09-01) see claims 1-10 | 5-15 |
| A | WO 2018140242 A1 (VANDERBILT UNIVERSITY) 02 August 2018 (2018-08-02) see claims 1-68 | 5-15 |
| A | US 2018251526 A1 (MEDIMMUNE LTD.) 06 September 2018 (2018-09-06) see claims 1-34 | 5-15 |
| A | WO 2019165019 A1 (VANDERBILT UNIVERSITY) 29 August 2019 (2019-08-29) see claims 1-90 | 5-15 |
| A | WO 2015010792 A1 (HUMABS BIOMED SA) 29 January 2015 (2015-01-29) see claims 1-52 | 5-15 |
| A | WO 2017005844 A1 (JANSSEN VACCINES & PREVENTION B. V.) 12 January 2017 (2017-01-12) see claims 1-13 | 5-15 |

☑ Further documents are listed in the continuation of Box C.   ☑ See patent family annex.

| * | Special categories of cited documents: |
| --- | --- |
| "A" | document defining the general state of the art which is not considered to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
| --- | --- |
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
| --- | --- |
| **20 December 2022** | **09 January 2023** |

| Name and mailing address of the ISA/CN | Authorized officer |
| --- | --- |
| **China National Intellectual Property Administration (ISA/ CN)** **No. 6, Xitucheng Road, Jimenqiao, Haidian District, Beijing 100088, China** | |
| Facsimile No. **(86-10)62019451** | Telephone No. |

Form PCT/ISA/210 (second sheet) (January 2015)

**INTERNATIONAL SEARCH REPORT**

| International application No. |
|---|
| **PCT/CN2022/123876** |

**C.    DOCUMENTS CONSIDERED TO BE RELEVANT**

| Category* | Citation of document, with indication, where appropriate, of the relevant passages | Relevant to claim No. |
|---|---|---|
| A | WO 2018075378 A1 (VANDERBILT UNIVERSITY) 26 April 2018 (2018-04-26) see claims 1-66 | 5-15 |

Form PCT/ISA/210 (second sheet) (January 2015)

**INTERNATIONAL SEARCH REPORT**

International application No.

**PCT/CN2022/123876**

Box No. I      Nucleotide and/or amino acid sequence(s) (Continuation of item 1.c of the first sheet)

1.  With regard to any nucleotide and/or amino acid sequence disclosed in the international application, the international search was carried out on the basis of a sequence listing:

   a.  ☑ forming part of the international application as filed:

      ☑ in the form of an Annex C/ST.25 text file.

      ☐ on paper or in the form of an image file.

   b.  ☐ furnished together with the international application under PCT Rule 13*ter*.1(a) for the purposes of international search only in the form of an Annex C/ST.25 text file.

   c.  ☐ furnished subsequent to the international filing date for the purposes of international search only:

      ☐ in the form of an Annex C/ST.25 text file (Rule 13*ter*.1(a)).

      ☐ on paper or in the form of an image file (Rule 13*ter*.1(b) and Administrative Instructions, Section 713).

2.  ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.

3.  Additional comments:

   [1]   The actually submitted sequence table is an XML file in Standard ST.26.

Form PCT/ISA/210 (continuation of first sheet) (January 2015)

**INTERNATIONAL SEARCH REPORT**

International application No.

**PCT/CN2022/123876**

| Box No. II | Observations where certain claims were found unsearchable (Continuation of item 2 of first sheet) |
|---|---|

This international search report has not been established in respect of certain claims under Article 17(2)(a) for the following reasons:

1. ☑ Claims Nos.: **15**
because they relate to subject matter not required to be searched by this Authority, namely:

[1]  PCT Rule 39.1(iv)-methods for the treatment of a human or animal body by surgery or therapy. The subject matter of claim 15 is reasonably expected to be: an application of the antibody, or antibody-drug conjugate or CAR-T cell expressing the antibody according to claims 1-7, or a combination thereof in the preparation of a drug for treating respiratory syncytial virus infection.

2. ☑ Claims Nos.: **1-4**
because they relate to parts of the international application that do not comply with the prescribed requirements to such an extent that no meaningful international search can be carried out, specifically:

[1]  Claims 1-4 comprise excessive options, variables, and possible permutations and combinations. For example, claim 1 comprises a large number of combinations of a different number of multiple light chain variable regions, mutations at locations, and a different number of multiple heavy chain variable regions, mutations at locations, and the light chain variable regions and the heavy chain variable regions. Therefore, claims 1-4 do not comply with PCT Article 6, to the extent that any meaningful search cannot be conducted.

3. ☐ Claims Nos.:
because they are dependent claims and are not drafted in accordance with the second and third sentences of Rule 6.4(a).

Form PCT/ISA/210 (continuation of first sheet) (January 2015)

<table>
<tr><td colspan="4" align="center"><b>INTERNATIONAL SEARCH REPORT</b><br>Information on patent family members</td><td colspan="2">International application No.<br><b>PCT/CN2022/123876</b></td></tr>
</table>

| Patent document cited in search report | | | Publication date (day/month/year) | Patent family member(s) | | | Publication date (day/month/year) |
|---|---|---|---|---|---|---|---|
| CN | 111606993 | A | 01 September 2020 | None | | | |
| WO | 2018140242 | A1 | 02 August 2018 | None | | | |
| US | 2018251526 | A1 | 06 September 2018 | CL | 2019002478 | A1 | 24 January 2020 |
| | | | | CA | 3051864 | A1 | 07 September 2018 |
| | | | | MX | 2019010408 | A | 19 December 2019 |
| | | | | IL | 268202 | A | 26 September 2019 |
| | | | | GB | 201802974 | D0 | 11 April 2018 |
| | | | | CO | 2019009034 | A2 | 30 August 2019 |
| | | | | TW | 202228779 | A | 01 August 2022 |
| | | | | KR | 20190125318 | A | 06 November 2019 |
| | | | | EC | SP19062769 | A | 30 September 2019 |
| | | | | PH | 12019501979 | A1 | 29 June 2020 |
| | | | | AU | 2018227502 | A1 | 15 August 2019 |
| | | | | US | 2021317191 | A1 | 14 October 2021 |
| | | | | EP | 3589648 | A1 | 08 January 2020 |
| | | | | SG | 11201906853 Q | A | 27 September 2019 |
| | | | | CR | 20190400 | A | 03 December 2019 |
| | | | | BR | 112019017755 | A2 | 07 April 2020 |
| | | | | JP | 2022095697 | A | 28 June 2022 |
| | | | | US | 2020339667 | A1 | 29 October 2020 |
| | | | | TW | 201840334 | A | 16 November 2018 |
| | | | | CN | 110418803 | A | 05 November 2019 |
| | | | | EA | 201991701 | A1 | 04 March 2020 |
| | | | | AR | 111083 | A1 | 29 May 2019 |
| | | | | KR | 20220020420 | A | 18 February 2022 |
| | | | | JP | 2020509031 | A | 26 March 2020 |
| | | | | WO | 2018160722 | A1 | 07 September 2018 |
| WO | 2019165019 | A1 | 29 August 2019 | None | | | |
| WO | 2015010792 | A1 | 29 January 2015 | US | 2019002538 | A1 | 03 January 2019 |
| | | | | US | 2016200802 | A1 | 14 July 2016 |
| | | | | US | 2021188949 | A1 | 24 June 2021 |
| | | | | US | 2019375825 | A1 | 12 December 2019 |
| WO | 2017005844 | A1 | 12 January 2017 | IL | 288541 | A | 01 February 2022 |
| | | | | CA | 2991002 | A1 | 12 January 2017 |
| | | | | JP | 2018520159 | A | 26 July 2018 |
| | | | | HK | 1250939 | A1 | 18 January 2019 |
| | | | | AU | 2016289492 | A1 | 01 March 2018 |
| | | | | US | 2018200360 | A1 | 19 July 2018 |
| | | | | IL | 256561 | A | 28 June 2018 |
| | | | | US | 2020061183 | A1 | 27 February 2020 |
| | | | | PL | 3319633 | T3 | 19 April 2021 |
| | | | | KR | 20180027560 | A | 14 March 2018 |
| | | | | EP | 3821906 | A1 | 19 May 2021 |
| | | | | DK | 3319633 | T3 | 01 February 2021 |
| | | | | EA | 201890220 | A1 | 29 June 2018 |
| | | | | CN | 107847580 | A | 27 March 2018 |
| | | | | EP | 3319633 | A1 | 16 May 2018 |
| | | | | ES | 2839880 | T3 | 06 July 2021 |
| | | | | ZA | 201800092 | B | 28 July 2021 |

Form PCT/ISA/210 (patent family annex) (January 2015)

INTERNATIONAL SEARCH REPORT
**Information on patent family members**

International application No.

**PCT/CN2022/123876**

| Patent document cited in search report | | | Publication date (day/month/year) | Patent family member(s) | | | Publication date (day/month/year) |
|---|---|---|---|---|---|---|---|
| | | | | BR | 112017027448 | A2 | 04 September 2018 |
| | | | | US | 2022125912 | A1 | 28 April 2022 |
| WO | 2018075378 | A1 | 26 April 2018 | US | 2019240316 | A1 | 08 August 2019 |
| | | | | EP | 3525820 | A1 | 21 August 2019 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- *J. biol. chem,* 1968, vol. 243, 3558
- **KABAT E.A et al.** Sequences of proteins of immunological interest. NIH Publication, 1991, 91-3242
- **SAMBROOK et al.** Molecular Cloning: Laboratory Manual. Cold Spring Harbor Laboratory Press, 1989
- **JASON S. MCLELLAN.** *Science,* 2013
- **DAVIDE CORTI.** *Nature,* 2013